# EUROPÄISCHE PATENTSCHRIFT

(11) **EP 2 723 330 B1**
(45) Veröffentlichungstag und Bekanntmachung des Hinweises auf die Patenterteilung: **08.07.2015**
(21) Anmeldenummer: 12750533.7
(22) Anmeldetag: 19.08.2012
(51) Int. Cl.: A61K 31/137, A61K 31/164, A61K 31/4174, A61K 31/4178, A61K 31/505, A61K 31/573, A61K 31/592, A61K 31/593, A61K 31/728, A61K 45/06, A61P 11/02, A61P 27/02

(54) **VASOKONSTRIKTORHALTIGES KOMBINATIONSTHERAPEUTIKUM**
VASOCONSTRICTOR-CONTAINING AGENT FOR COMBINATION THERAPY
AGENT DE POLYTHÉRAPIE CONTENANT DES VASOCONSTRICTEURS

(30) Priorität: 19.08.2011 DE 102011111111
(43) Veröffentlichungstag der Anmeldung: 30.04.2014
(73) Patentinhaber: Maria Clementine Martin Klosterfrau Vertriebsgesellschaft mbH, 50670 Köln (DE)
(72) Erfinder: GREVE, Harald, 40545 Düsseldorf (DE)
(74) Vertreter: Strehlke, Ingo Kurt
(86) Internationale Anmeldenummer: PCT/EP2012/003524
(87) Internationale Veröffentlichungsnummer: WO 2013/026557

(56) Entgegenhaltungen:
- EP-A1- 0 992 242
- EP-A2- 1 532 986
- WO-A1-2007/039322
- WO-A1-2009/032887
- DE-A1- 19 541 919
- DE-A1-102008 036 725
- MCNALLY P ET AL: "Vitamin D receptor agonists inhibit pro-inflammatory cytokine production from the respiratory epithelium in cystic fibrosis", JOURNAL OF CYSTIC FIBROSIS, ELSEVIER, NL, Bd. 10, Nr. 6, 28. Juni 2011 (2011-06-28), Seiten 428-434, XP028114935, ISSN: 1569-1993, DOI: 10.1016/J.JCF.2011.06.013 [gefunden am 2011-07-05]

## Beschreibung

Die vorliegende Erfindung betrifft das medizinische Gebiet der prophylaktischen bzw. therapeutischen Behandlung von insbesondere entzündlichen Erkrankungen der oberen Atemwege.

Insbesondere betrifft die vorliegende Erfindung ein Kombinationstherapeutikum, welches sich zur Verwendung bei der prophylaktischen bzw. therapeutischen topischen Behandlung von entzündlichen Erkrankungen der Nase und/oder der Nasennebenhöhlen eignet.

Weiterhin betrifft die vorliegende Erfindung eine Applikationsvorrichtung, welche das Kombinationstherapeutikum nach der Erfindung enthält.

Unter einer Rhinitis (synonym gelegentlich auch als Nasenkatarrh, Schnupfen oder Koryza bezeichnet) versteht man im Rahmen der vorliegenden Erfindung insbesondere eine akute oder chronische Entzündung der Nasenschleimhäute, wobei die Rhinitis insbesondere infektiösen, allergischen oder pseudoallergischen Ursprungs sein kann. Am häufigsten tritt eine Rhinitis im Rahmen einer sogenannten Erkältung auf.

Neben einer Unterscheidung von akuter Rhinitis einerseits und chronischer Rhinitis andererseits unterscheidet man auch verschiedene Formen der Rhinitis, beispielsweise die *Rhinitis acuta, Rhinitis atrophicans, Rhinitis allergica, Rhinitis hypertrophica, Rhinitis medicamentosa, Rhinitis pseudo-membranacea, Rhinitis sicca* und *Rhinitis vasomotorica.*

Bei der sogenannten akuten Rhinitis (*Rhinitis acuta*), d. h. dem gewöhnlichen Schnupfen, handelt es sich in der Regel um einen im Allgemeinen harmlosen Infekt der Nasenschleimhäute und damit um eine infektiöse Rhinitis, welche durch eine Vielzahl von Viren (insbesondere Rhinoviren und/oder Adenoviren) ausgelöst werden kann; Hauptmerkmal einer akuten Rhinitis ist eine sogenannte "laufende Nase" und eine Verstopfung der Nase durch die Anschwellung der Schleimhäute.

Insgesamt sind mehr als 200 "Schnupfenviren" als mögliche Auslöser einer viralen Rhinitis bekannt, wie sie üblicherweise im Rahmen einer gewöhnlichen Erkältung auftreten kann. Im Rahmen einer Erkältung, welche oftmals mit einer Rhinitis beginnt, verschwindet die *Rhinitis acuta* aber im Allgemeinen nach einem bestimmten Zeitraum. Gelegentlich kann es jedoch mitunter auch zu einer Chronifizierung kommen, welche oftmals mit einer Volumenzunahme der Schleimhäute unter anderem im Bereich der Nasenmuscheln mit Behinderung der Nasenatmung einhergeht.

Die *Rhinitis allergica* (allergischer Schnupfen) stellt eine Erkrankung des atopischen Formenkreises dar. Diese Krankheiten betreffen insbesondere durch Abwehrmoleküle, wie Immunglobulin E, des Körpers hervorgerufene Entzündungen. Die *Rhinitis allergica* betrifft die oberen Luft- bzw. Atemwege und geht mit einer hohen Zahl an Begleiterkrankungen einher. So kann sie zu anderen Atemwegserkrankungen, wie Asthma und Sinusitis, führten. Die Erkrankung beginnt meistens im frühen Kindesalter und führt oftmals zu einer nachhaltigen Beeinträchtigung der Lebensqualität. Die gesundheitlichen Auswirkungen betreffen das Sozialleben, die schulische Leistungsfähigkeit und die Arbeitsproduktivität. Im Erwachsenenalter spielen zudem Kreuzallergien eine große Rolle.

Für weitergehende Einzelheiten zum Begriff der Rhinitis kann insbesondere verwiesen werden auf Pschyrembel, Medizinisches Wörterbuch, 257. Auflage, Seiten 1331/1332, insbesondere Stichwörter: *"Rhinitis", "Rhinitis allergica", "Rhinitis atrophicans", "Rhinitis hyperplastica", "Rhinitis pseudomembranacea", "Rhinitis sicca"* und *"Rhinitis vasomotorica".*

Da es also eine Vielzahl verschiedener Typen von Viren gibt, welche eine Rhinitis auslösen können, und da eine Vielzahl von Ursachen für das Auftreten einer Rhinitis existiert, können Rhinitiden, insbesondere akute Rhinitiden, im Allgemeinen nicht kausal, sondern nur symptomatisch, vorzugsweise topisch, behandelt werden, zumeist unter Anwendung von Sympathomimetika (synonym auch als sogenannte "Abschweller" oder "Dekongestiva" bezeichnet), vorzugweise alpha-Sympathomimetika, wie Xylometazolin und Oxymetazolin oder deren physiologisch verträgliche Salze.

Diese Sympathomimetika führen aufgrund ihrer vasokonstriktorischen Eigenschaften nach lokaler bzw. topischer Anwendung in der Nase zwar zu einer Nasenschleimhautabschwellung, bewirken jedoch bei wiederholten Anwendungen oftmals eine Austrocknung der Nasenschleimhäute, einhergehend mit entzündlichen Irritationen der Nasenschleimhäute - was nicht selten zu einer erhöhten Infektionsgefahr führt, da die Nasenschleimhäute im ausgetrockneten und entzündeten Zustand nicht mehr ihre Schutz- und Filterfunktionen im vollen Umfang aufrechterhalten können und folglich Krankheitserreger ungehindert in die Atemwege gelangen können.

Sympathomimetika, insbesondere in Form von alpha-Sympathomimetika, werden oftmals auch zur Behandlung von entzündlichen Erkrankungen des Auges eingesetzt, insbesondere auch bei Konjunktivitis bzw. einer Augenbindehautentzündung. In diesem Zusammenhang kann der Einsatz von Sympathomimetika bzw. Vasokonstriktoren die Reizung der Augen lindern, insbesondere durch eine durch den Vasokonstriktor induzierte Verengung der Blutgefäße, wodurch eine mit der Konjunktivitis einhergehende Augenrötung zurückgeht. Jedoch kann die Therapie einer Konjunktivitis auf Basis von Vasokonstriktoren bzw. Sympathomimetika insbesondere bei längerer Therapiedauer bzw. hoher Dosierung des Vasokonstriktors zu einem erhöhten Augeninnendruck bzw. zu einem sogenannten "trockenen Auge" führen.

Um die Wirkung von Vasokonstriktoren insbesondere im Hinblick auf die Therapie von entzündlichen Erkrankungen des Auges, insbesondere Konjunktivitis, zu erhöhen, werden im Stand der Technik beispielsweise Zinksulfate in Kombination mit Vasokonstriktoren eingesetzt, was jedoch mitunter nicht zu einer nachhaltigen Steigerung der Wirkeffizienz führt und zudem mit weiteren Nebenwirkungen verbunden ist, insbesondere was die Verträglichkeit der diesbezüglichen Therapeutika anbelangt.

Grundsätzlich kommt der Einsatz von Vasokonstriktoren bzw. Sympathomimetika auch im Hinblick auf die Therapie einer Uveitis und somit einer Entzündung der mittleren Augenhaut in Betracht.

Für weitergehende Einzelheiten zu den Begriffen "Konjunktivitis" und "Uveitis" kann insbesondere verwiesen werden auf Pschyrembel, Medizinisches Wörterbuch, 275. Auflage, Seite 810, Stichwort: *"Konjunktivitis"* sowie Seite 1605, Stichwort: *"Uveitis".*

Insbesondere ist die Verwendung von Vasokonstriktoren bzw. Symphatomimetika auch insofern nachteilig, als insbesondere bei längerem Anwendungsverlauf bzw. höheren Dosierungen mitunter auch systemische Nebenwirkungen auftreten können. Zudem fokussiert der therapeutische Einsatz von Vasokonstriktoren vorrangig auf eine symptomatische Behandlung, wobei die Wirkeffizienz in Bezug auf die zugrundeliegende Erkrankung nicht immer optimal ist bzw. der Vasokonstriktor selbst zu nachteiligen Effekten führt, woraus längere Therapieverläufe bei höherer Dosierung resultieren können.

Die WO 2009/032887 A1 betrifft ein Verfahren zur Behandlung bzw. Verhütung einer Atemwegsinfektion, wobei hierzu Cholecalciferol verabreicht werden sollen.

Weiterhin betrifft die auf die EP 1 532 986 A2 eine zur Behandlung von Rhinitiden geeignete pharmazeutische Zusammensetzung, welche eine spezielle Kombination eines Sympathomimetikums mit vasokonstriktorischer bzw. schleimhautabschwellender Wirkung und ein vorzugsweise saures Glykosaminoglykan enthält.

Weiterhin betrifft die DE 195 41 919 A1 eine pharmazeutische Zubereitung zur Behandlung akuter Rhinitiden, welche in Kombination und in physiologischer Konzentration ein zur topischen Anwendung geeignetes Sympathomimetikum mit 2-Imidazolin-Struktur einerseits sowie Panthotenol bzw. Pantothensäure andererseits umfasst.

Die EP 0 992 242 A1 betrifft eine ophthalmische Zusammensetzung zur Prophylaxe bzw. Behandlung von Keratokonjunktivitis sicca und somit des sogenannten trockenen Auges, wobei die dort beschriebene Zusammensetzung als Wirksubstanz Vitamin D bzw. aktives Vitamin D bzw. Vitamin D-Analoga aufweisen soll.

Darüber hinaus betrifft die WO 2007/039322 A1 eine Vitamin D-Zusammensetzung zur Behandlung von Uveitis und somit einer Entzündung der mittleren Augenhaut.

Weiterhin betrifft die wissenschaftliche Publikation gemäß McNally et al., "Vitamin D receptor agonists inhibit pro-inflammatory cytokine production from the respiratory epitheium in cystic fibrosis", Journal of Cystic Fibrosis, Nr. 10, 2011, Seiten 428 bis 434, wissenschaftliche Untersuchungen zur Wirksamkeit von Vitamin D-Rezeptoragonisten im Hinblick auf die Behandlung von zystischer Fibrose und somit von Mucoviszidose.

Schließlich betrifft die DE 10 2008 036 725 A1 eine pharmazeutische Zubereitung zur prophylaktischen bzw. kurativen Behandlung von trockener Nasenschleimhaut bzw. Rhinitiden, wobei die Zusammensetzung in Kombination Ectoin bzw. ein Ectoinderivat einerseits und Pantothenol bzw. Pantothensäure andererseits umfassen soll.

Die der vorliegenden Erfindung zugrundeliegende Aufgabe besteht daher in der Bereitstellung eines bzw. einer für die topische, insbesondere nasale, bevorzugt intranasale Applikation, insbesondere für die Behandlung von Rhinitiden geeigneten Kombinationstherapeutikums bzw. Zusammensetzung, welche die zuvor geschilderten Nachteile des Standes der Technik zumindest weitgehend vermeidet oder aber wenigstens abschwächt.

Insbesondere besteht eine Aufgabe der vorliegenden Erfindung darin, ein entsprechendes Kombinationstherapeutikum bereitzustellen, welches im Hinblick auf die zugrundeliegenden Indikationen, insbesondere in Bezug auf die Behandlung von insbesondere entzündlichen Erkrankungen der oberen Atemwege, vorzugsweise der Nase bzw. der Nasennebenhöhlen, wie Rhinitis, über eine verbesserte Wirkeffizienz verfügt und infolgedessen einen verbesserten Therapieverlauf unter Minimierung bzw. Dosisreduktion und besserer Verträglichkeit der eingesetzten therapeutisch aktiven Substanzen ermöglicht.

Insbesondere soll im Rahmen der vorliegenden Erfindung eine verbesserte und/oder effizientere Therapie zur Behandlung von entzündlichen Erkrankungen der oberen Atemwege bereitgestellt werden.

Insbesondere soll im Rahmen der vorliegenden Erfindung eine verbesserte Wirkeffizienz bzw. ein breiteres Anwendungsspektrum von insbesondere lokal bzw. topisch, insbesondere nasal applizierbarer Therapeutika (wie z. B. von Kortikosteroiden, Antihistaminika, Antiallergika etc.) ermöglicht bzw. erreicht werden.

Zur Lösung der zuvor gestellten Aufgabe schlägt die vorliegende Erfindung - gemäß einem ersten Aspekt der vorliegenden Erfindung - ein Kombinationstherapeutikum, insbesondere pharmazeutisches Kombinationstherapeutikum, nach Anspruch 1 vor; weitere, insbesondere vorteilhafte Ausgestaltungen des erfindungsgemäßen Kombinationstherapeutikums sind Gegenstand der diesbezüglichen Unteransprüche.

Schließlich betrifft die vorliegende Erfindung - gemäß einem wiederum weiteren Aspekt der vorliegenden Erfindung - die erfindungsgemäße Applikationsvorrichtung gemäß Anspruch 13, wobei die Applikationsvorrichtung das Kombinationstherapeutikum nach der Erfindung enthält. Weitere, insbesondere vorteilhafte Ausgestaltungen der erfindungsgemäßen Applikationsvorrichtung sind Gegenstand der diesbezüglichen Unteransprüche.

Es versteht sich von selbst, dass im Folgenden besondere Ausgestaltungen, Ausführungsformen, Vorteile und dergleichen, welche nachfolgend zu Zwecken der Vermeidung von Wiederholungen nur zu einem Erfindungsaspekt beschrieben sind, auch in Bezug auf die übrigen Erfindungsaspekte entsprechend gelten, ohne dass dies einer ausdrücklichen Erwähnung bedarf.

Bei sämtlichen nachstehend genannten relativen bzw. prozentualen gewichtsbezogenen Angaben, insbesondere Mengenangaben, ist weiterhin zu beachten, dass diese im Rahmen der vorliegenden Erfindung vom Fachmann derart auszuwählen sind, dass in der Summe der jeweiligen Inhaltsstoffe, Wirkstoffe, Zusatz- bzw. Hilfsstoffe oder dergleichen stets 100 % bzw. 100 Gew.-% resultieren. Dies versteht sich für den Fachmann aber von selbst.

Im Übrigen gilt, dass der Fachmann anwendungsbezogen oder einzelfallbedingt von den nachfolgend aufgeführten Zahlen-, Bereichs- oder Mengenangaben abweichen kann, ohne dass er den Rahmen der vorliegenden Erfindung verlässt.

Zudem gilt, dass sämtliche im Folgenden genannten Werte- bzw. Parameterangaben oder dergleichen grundsätzlich mit genormten bzw. standardisierten oder explizit angegebenen Bestimmungsverfahren oder anderenfalls mit dem Fachmann auf diesem Gebiet an sich geläufigen Bestimmungs- bzw. Messmethoden ermittelt bzw. bestimmt werden können.

Dies vorausgeschickt, wird die vorliegende Erfindung nachfolgend im Detail erläutert.

Die Anmelderin hat nun in völlig überraschender Weise herausgefunden, dass die Nachteile des Standes der Technik dadurch überwunden werden können, dass erfindungsgemäß ein Kombinationstherapeutikum, insbesondere pharmazeutisches Kombinationstherapeutikum, zur Verwendung bei der prophylaktischen und/oder therapeutischen topischen Behandlung von entzündlichen Erkrankungen der Nase und/oder der Nasennebenhöhlen in Form von Rhinitis, Rhinosinusitis bzw. Sinusitis bereitgestellt wird. Das erfindungsgemäße Kombinationstherapeutikum zeichnet sich auch dadurch aus, dass das Kombinationstherapeutikum mindestens einen Vitamin D-Rezeptoragonisten (VDA) einerseits und mindestens einen Vasokonstriktor in Form eines alpha-Sympathomimetikums, vorzugsweise alpha-1-Sympathomimetikums, oder dessen physiologisch unbedenkliche Salze andererseits umfasst.

Denn die Anmelderin hat in völlig überraschender Weise gefunden, dass auf Basis der erfindungsgemäßen Kombination eine synergistische Wirkverstärkung durch den Vitamin D-Rezeptoragonisten (VDA) auch in Bezug auf den Vasokonstrikor bzw. das alpha-Sympathomimetikum vorliegt und auf dieser Basis ein wirkeffizientes Kombinationstherapeutikum für die angeführten Indikationen bereitgestellt wird, welches zu einem verbesserten Therapieerfolg sowie zu einem schnelleren Abklingen der Erkrankung führt bzw. eine Dosisreduktion des Vasokonstrikors ermöglicht. Darüber hinaus resultiert im Rahmen der Anwendung des erfindungsgemäßen Kombinationstherapeutikums auch eine durch den Vitamin D-Rezeptoragonisten induzierte synergistische Wirkverstärkung etwaiger weiterer Wirkstoffe, wie insbesondere Kortikosteroiden. In diesem Zusammenhang kommt auch eine weiterführende Kombination des erfindungsgemäßen Kombinationstherapeutikums insbesondere mit sogenannten Leitlinientherapeutika für die jeweils zugrundeliegende Erkrankung bzw. Indikation in Betracht, wobei es im Rahmen der vorliegenden Erfindung in völlig unerwarteter Weise gelungen ist, die Wirkeffizienz der jeweils eingesetzten Therapeutika nachhaltig zu verbessern, gleichermaßen einhergehend mit der Möglichkeit einer Dosisreduktion bzw. Verkürzung des Behandlungszeitraums. In diesem Zusammenhang kommt dem erfindungsgemäßen Kombinationstherapeutikum insbesondere auch eine verbesserte antientzündliche Wirkung zu.

Der im Rahmen der vorliegenden Erfindung völlig überraschend aufgefundene und durch die nachfolgenden Ausführungsbeispiele hinsichtlich der aufgefunden Wirkverstärkung bzw. Steigerung der Wirkeffizienz belegte synergistische Effekt, welcher mit dem erfindungsgemäßen Kombinationstherapeutikums einhergeht, kann insbesondere - ohne sich auf diese Theorie beschränken zu wollen - darauf zurückgeführt werden, dass die Expression des auch für entzündungshemmende bzw. antientzündliche Prozesse relevanten Vitamin D-Rezeptors durch Vitamin D bzw. durch den Vitamin D-Rezeptoragonisten (VDA) selbst reguliert wird. Demnach ist der Vitamin D-Rezeptor bei Vitamin D-Mangelzuständen, welche oftmals bei den zugrundeliegenden Erkrankungen vorliegen, herunterreguliert bzw. kann durch Vitamin D hochreguliert werden. Die therapeutische Verabreichung von Vitamin D bzw. des in Rede stehenden Vitamin D-Rezeptoragonisten (VDA) bewirkt somit im Rahmen der vorliegenden Erfindung - ohne sich auf diese Theorie beschränken zu wollen - insbesondere in den therapeutisch angeführten Mengen eine Hochregulation bzw. verstärkte Expression des Vitamin D-Rezeptors, welche - gleichermaßen ohne sich dabei auf diese Theorie beschränken zu wollen - eine etwaige durch das alpha-Sympathomimetikum hervorgerufene Aktivierung bzw. Stimulation von alpha-2-Andrenorezeptoren mit gegebenenfalls folgender Stimulation der Prostaglandinsynthese, insbesondere von PGE2, überkompensiert. Insbesondere hat die Anmelderin nunmehr in völlig überraschender Weise jedoch gefunden, dass die erfindungsgemäß eingesetzten Vasokonstriktoren, insbesondere Xylometazolin, selbst über eine antientzündliche Wirkung verfügen, welche - weiterhin völlig überraschend - durch den Vitamin D-Rezeptoragonisten synergistisch verstärkt wird bzw. *vice versa.* Zudem wird verhindert, dass - gleichermaßen ohne sich dabei auf diese Theorie beschränken zu wollen - einem aufgrund der hemmenden Wirkung von COX II-Metaboliten, wie Prostaglandin, auf den Vitamin D-Rezeptor sinkende Vitamin D-Wirkung bzw. abnehmenden Vitamin D-Spiegel im Körper effektiv entgegengewirkt und dieser sogar überkompensiert wird. Zudem hemmt Vitamin D bzw. der Vitamin D-Rezeptoragonist - gleichermaßen ohne sich auf diese Theorie beschränken zu wollen - sowohl das Protein MKP-1 als auch p-38-MAPK. Insofern kann Vitamin D auch zu einer Beeinflussung insbesondere des MAP-Kinase-Signaltransduktionsweg führen, welcher unter anderem entzündliche Prozesse im Körper induzieren kann.

Durch die gegebenenfalls vorgesehene weiterführende Kombination bzw. Co-Medikation mit Steroiden, insbesondere Kortikosteroiden, beispielsweise bei *Rhinitis allergica,* wird - gleichermaßen ohne sich auf diese Theorie beschränken zu wollen - aufgrund der Steroidgabe eine Verminderung der Prostaglandinsynthese neben einer Hochregulation von Steroidrezeptoren und der Phosphatase MKP-1, welche im Allgemeinen für immunmodulatorische Prozesse eine Rolle spielt, erzielt, was einen weiteren positiven Einfluss auf die Verringerung entzündlicher Prozesse hat.

Auf Basis der gezielten Wirkstoffkombination wird somit in völlig unerwarteter Weise ein synergistischer Effekt hinsichtlich der Wirkeffizienz des erfindungsgemäßen Kombinationstherapeutikums bereitgestellt.

Üblicherweise sind gesunde Personen bei ausreichender Sonnenexposition grundsätzlich unabhängig von einer zusätzlichen Aufnahme von Vitamin D über die Nahrung, da unter Sonneneinfluss bzw. UV-Strahlung ausgehend von der Vorläufersubstanz von Vitamin D, nämlich 7-Dehydroxycholesterol, in der Haut Prävitamin D₃ entsteht. Aus dem instabilen Prävitamin D₃ entsteht nach etwa 48 Stunden Calcitriol, welches eine physiologisch aktive Form von Vitamin D₃ darstellt. Darüber hinaus können ausgehend von 7-Dehydroxycholesterol zum Schutz vor einer Vitamin D₃-Überproduktion Lumisterol und Tachysterol gebildet werden. Ein Mangel oder das Meiden von Sonne aber auch bestimmte Erkrankungen erfordern mitunter jedoch eine nahrungsabhängige Aufnahme von pflanzlichem Vitamin D₂ bzw. Ergocalciferol bzw. von tierischem Vitamin D₃ bzw. Cholecalciferol.

Da im Körper die aktive Form von Vitamin D erst durch enzymatische Prozesse bereitgestellt wird, stellt der körpereigene Vitamin D-Metabolismus ein komplexes Netzwerk auf Basis zahlreicher aufeinanderfolgender Stoffwechselschritte dar. Im Rahmen des Vitamin D-Metabolismus erreichen die verschiedenen Vitamin D-Metabolite, insbesondere gebunden an das Vitamin D-bindende Protein (VDB), die Leber oder werden im Fettgewebe aufgrund ihrer Lipophilie gespeichert. In der Leber werden Vitamin D-Metabolite durch die 25-Hydroxylase (CYP2R1) zu Calcidiol (25-Hydroxyvitamin D bzw. 25-OH D) mit höherer Bindungsaffinität zu dem Vitamin D-Rezeptor (synonym auch als Vitamin D₃-Rezeptor bezeichnet) und mit einer Halbwertszeit von etwa 2,5 Wochen metabolisiert. Die Konversionsrate kann dabei 0,1 bis 0,2. %/10⁶ Zellen/Stunde betragen. Der Spiegel von Calcidiol entspricht der sonnen- und/oder diätbedingten Vitamin D₃-Menge (100 bis 200 IU/Tag) und nimmt im Sommer im Mittel um 12 ng/ml zu.

Durch die 1-alpha-Hydroxylase entsteht im Rahmen des Vitamin D-Metabolismus schließlich der aktive Metabolit Calcitriol (1,25-Dihydroxyvitamin D bzw. 1,25-DOH D) in der Niere und anderen Zellen. Calcitriol besitzt im Vergleich zu Calcidiol mitunter ein stärkeres Potential zur Hypercalcämie. Die Aktivität der 1-alpha-Hydroxylase und somit die renale Bildung von Calcitriol wird durch verschiedene Hormone, insbesondere Parathormon, Oestrogene, Calcitonin, Wachstumshormone und Prolaktin, sowie durch ein Absinken des Phosphatspiegels, insbesondere Hypophosphatämie, stimuliert. Durch Calcium und Calcitriol selber hingegen wird die 1-alpha-Hydroxylase gehemmt. Neben den Zellen der Niere wird die 1-alpha-Hydroxylase auch in anderen Zellen, wie Makrophagen oder Granulomen, exprimiert. Im Gegensatz zu den Zellen der Nieren haben in den übrigen Zellen jedoch Parathormon, Calcium und Vitamin D-Metabolite keinen Einfluss auf deren Aktivität. Nebenwirkungen von Glukokortikoiden, Chloroquin oder Ketoconazol hingegen sind allgemein mit einer Reduktion von Calcitriol verbunden. Bei Adipositas liegt häufig ein Mangel an Calcidiol aufgrund seiner Lipophilie vor, wobei dennoch zwischen Ursache und Folge des Vitamin D-Mangels auch bei Adipositas nicht sicher differenziert werden kann.

Vitamin D-Rezeptoren (VDR) werden von verschiedenen Zellen exprimiert, beispielsweise von Monozyten, Makrophagen, Thrombozyten, Lymphozyten sowie auch Epithelzellen. Folglich kommen Vitamin D-Rezeptoren (VDR) unter anderem in den Atemwegen sowie im Darm vor. Die Expression des Vitamin D-Rezeptors (VDR) für Calcitriol wird dabei durch Calcitriol selbst kontrolliert. Insbesondere ist ein übermäßiger Anstieg der Calcitriolkonzentration mitunter mit einer Hemmung bzw. Verringerung der Expression von Vitamin D-Rezeptoren (VDR) verbunden. Liegt Calcitriol in einer Konzentration von 10⁻⁷ mol/l vor, erfolgt eine 9-fache Expression, wohingegen die Anwesenheit einer höheren Calcitriol-Konzentration von 10⁻⁶ mol/l lediglich zu einer 1,6-fachen Expression von VDR führt.

Im Gegensatz dazu werden Vitamin D-Rezeptoren (VDR) für Calcidiol weder durch Calcidiol selbst noch durch Calcitriol herunterreguliert, was insbesondere im Hinblick auf den therapeutischen Einsatz von Calcitriol bzw. Calcidiol zu berücksichtigen ist.

Insgesamt ist die Rolle dieser Rezeptoren weitgehend unerforscht. Unter anderem wird von einer Involvierung in die Zunahme der intrazellulären CalciumKonzentration, die Phospolipase C-Aktivität, die Regulation der DNA-Transkription und die RNA-Synthese mit einer höheren Bindungsaffinität für Calcitriol im Vergleich zu Calcidiol vermutet. In verschiedenen Zellmodellen wurde zudem eine zunehmende Zelldifferenzierung und eine Hemmung der Zellproliferation durch Calcitriol beschrieben und es existieren Hinweise, dass auch Calcidiol hierbei eine Rolle spielt.

Als ungefährer Tagesbedarf von Vitamin D bzw. Vitamin D₃ werden 600 IU empfohlen und bei älteren Patienten oder bei erhöhtem Osteoporoserisiko 800 IU bzw. 4.000 IU als maximale tägliche Aufnahme, um einen Calcidiol-Spiegel von mindestens 20 ng/ml zu erreichen. Andere Empfehlungen zur Behandlung der Osteoporose gehen von einer täglichen Aufnahme von 2.000 IU aus, um einen Plasmaspiegel von > 30 ng/ml sicherzustellen. Hierbei mangelt es jedoch an gesicherten Erfahrungen, um die Entwicklung verschiedener Erkrankungen bei Überdosierung bis hin zur Arteriosklerose und Bauchspeicheldrüsenkarzinomen zu vermeiden. Das *US-Institute of Medicine* empfiehlt in diesem Zasammenhang eine orale Calcitriol-Aufnahme von 50.000 IU/Woche über einen Zeitraum von acht Wochen. Anschließend erfolgt eine Senkung der Calcitriol-Zufuhr auf 50.000 IU alle zwei bis vier Wochen. Alternativ wird die Gabe von 100.000 IU über einen Zeitraum von drei Monaten angeführt.

Insgesamt existiert eine Vielzahl von Erkrankungen, welche mit einem Vitamin D-Mangel assoziiert sind. Dazu gehören überwiegend chronische Erkrankungen, wie verschiedene Tumorerkrankungen, Autoimmunerkrankungen, sowie Infektionserkrankungen, beispielsweise Tuberkulose, kardiovaskuläre Erkrankungen, wie die koronare Herzerkrankung und Bluthochdruck, *Morbus Crohn, Colitis ulcerosa,* Arthritis, Psoriasis und Nierenerkrankungen. Auch Behandlungen mit bestimmten Therapeutika, insbesondere Kortikosteroiden und Antiepileptika, führen zu einem Vitamin D bzw. Vitamin D₃-Mangel als Nebenwirkung. Weiterhin können auch psychische Erkrankungen, wie Depressionen oder Schizophrenie, mit einem Vitamin D bzw. Vitamin D₃-Mangel assoziiert sein. Bei allen vorgenannten Erkrankungen kann im Allgemeinen ein Mangel an Calcitriol nachgewiesen werden, wobei bislang nicht geklärt ist, ob der Mangel an Vitamin D bzw. Vitamin D₃ die Ursache oder die Folge der Erkrankung ist.

Ein Mangelzustand an Calcitriol wird üblicherweise durch gleichzeitige Bestimmung des Präkursors oder Metaboliten Calcidiol bestimmt.

Es wird angenommen, dass - ohne sich auf diese Theorie beschränken zu wollen - der Wirkmechanismus von Vitamin D auf einer Bindung von Calcidiol bzw. Calcitriol an den Vitamin D-Rezeptor (VDR) basiert. Dieser Komplex bindet an das Vitamin D-Response-E-lement in der Promotorregion des Vitamin D-Responsegenes, so dass es zu einer Zunahme der durch die RNA-Polymerase vermittelten Transkription des Vitamin D-Responsegenes kommt. Infolge der verstärkten Transkription des Vitamin D-Responsegenes werden verschiedene und immunrelevante Vorgänge vermittelt bzw. induziert, unter anderem die Aktivierung der CD4-Rezeptoren von T-Helferzellen, die Hemmung der von T-Helferzellen produzierten Zytokinen (Interferon-gamma, Interleukin-2, Interleukin-5), welche insbesondere zu Entzündungsreaktionen führen, und eine Stimulation von Interleukin-4. Darüber hinaus kann auch eine Hemmung von Interferon-gamma bei gleichzeitiger Stimulation von Interleukin-4, Interleukin-5 und Interleukin-10 auftreten. Auch kann eine Hemmung der Interleukin-12 stimulierten Produktion von Interferon-gamma sowie eine Hemmung von Interleukin-4 und Interleukin-13 durch Calcitriol erfolgen. Aus den vorstehenden Ausführungen ist ersichtlich, dass Vitamin D, insbesondere Calcitriol, an verschiedenen Immunantworten beteiligt ist.

Darüber hinaus wird auch angenommen, dass Calcidiol eine Rolle für Prozesse des Immunsystems haben könnte. Im Rahmen der vorliegenden Erfindung wurde gefunden, dass Calcidiol und Calcitriol die Produktion des im Zusammenhang mit Entzündungsreaktionen gebildeten Tumomekrosefaktor-alpha (TNF-alpha) hemmen, wobei Calcidiol die TNF-alpha-Bildung bis zu dreimal stärker hemmt als Calcitriol und somit überraschenderweise eine nochmals stärkere Entzündungshemmung bewirkt. Darüber hinaus wurde überraschenderweise gefunden, dass auch Calcidiol in synergistischer Weise mit verschiedenen Leitlinientherapeutika interagiert und diese in ihrer Wirkung verstärkt. Für Calcitriol ist in diesem Zusammenhang beachtlich, dass Calcitriol in höheren Konzentrationen mitunter eine Downregulation von Vitamin D-Rezeptoren (VDR) induziert, so dass die Wirkung von Calcitriol insbesondere bei höheren Konzentrationen von Calcitriol abgeschwächt sein kann. Dennoch kommt auch Calcitriol im Rahmen der vorliegenden Erfindung eine hohe Bedeutung zur Behandlung der zugrundeliegenden Erkrankungen zu.

Auf dieser Grundlage wird im Rahmen der vorliegenden Erfindung auch eine Co-Medikation von Vitamin D bzw. Vitamin D-Metaboliten, insbesondere Calcidiol und/oder Calcitriol, vorzugsweise Calcidiol, in Kombination mit verschiedenen entzündungshemmenden und/oder antiallergischen Leitlinientherapeutika, wie nachfolgend definiert, vorgeschlagen.

Im Rahmen der vorliegenden Erfindung steht vorrangig die entzündungshemmende Wirkung von Vitamin D im Vordergrund. Erfindungsgemäß von Bedeutung ist dabei einerseits die entzündungshemmende Wirkung von Vitamin D an sich, welche in völlig überraschender Weise im Rahmen eines synergistischen, antientzündlichen Effektes durch eine Kombination mit weiteren Wirkstoffen gesteigert werden kann. Im Rahmen der vorliegenden Erfindung wird in diesem Zusammenhang insbesondere eine Art Zusatztherapie bzw. Co-Medikation zur Verbesserung der klinischen Effekte bzw. eine Reduktion des Medikamentenbedarfs auch im Rahmen von Leitlinientherapien bereitgestellt.

Was im Speziellen den Zusammenhang zwischen Autoimmunerkrankungen und Vitamin D-Mangel anbelangt, wird vermutet, dass bei verschiedenen Autoimmunerkrankungen ein niedriger Vitamin D-Spiegel durch eine chronische, intrazelluläre Infektion verursacht wird, welche zu einer Fehlfunktion des Vitamin D-Rezeptors führt und auf diese Weise wiederum die Empfänglichkeit für sekundär auftretende Infektionen erhöht. Dies wird insbesondere für die Krankheiten *Psoriasis vulgaris, Lupus erythematosis,* rheumatoide Arthritis, Sarkoidose, Uveitis und weitere vermutet. Weiterhin sind Patienten mit chronisch polypoider Rhinosinusitis und einer Pilz-allergischen Rhinosinusitis teilweise von Vitamin D-Mangelzuständen betroffen, welche auch mit Nasenpolypen, einer vermehrten ossären Erosion und einer Verminderung von dendritischen Zellen assoziiert sind. Häufig tritt ein Vitamin D-Mangel auch bei verschiedenen Formen chronisch-entzündlichen Darmerkrankungen (IBD), insbesondere bei *Morbus Crohn* (CD) und *Colitis ulcerosa* (UC), auf. Erfindungsgemäß wurde in völlig überraschender Weise gefunden, dass auf Basis des erfindungsgemäßen Kombinationstherapeutikums nicht nur eine effiziente Wirksteigerung bzw. Dosisreduzierung von weiteren Wirkstoffen, wie sie beispielsweise im Rahmen von Leitlinientherapien eingesetzt werden, erreicht werden kann, sondern dass auch den zuvor mit den zugrundeliegenden Krankheiten aufgefundenen Vitamin D-Mangelzuständen in effektiver Weise entgegengetreten werden kann, was einen weiteren zentralen Vorteil der vorliegenden Erfindung darstellt.

Im Stand der Technik wird jedoch im Zusammenhang mit Vitamin D-Mangelerkrankungen, welche vorwiegend aus Beobachtungsstudien und teilweise aus Interventionsstudien bei malignen Erkrankungen, wie Colonkarzinomen, stammen, zunehmend Empfehlungen nationaler und internationaler Fachverbände ausgesprochen, den Vitamin D-Spiegel nicht routinemäßig zu bestimmen. Insbesondere wird von unnötigen Vitamin D-Supplementierungen abgeraten. So empfiehlt das *U.S. Institute of Medicine* (IOM) im Januar 2011, dass für die Knochengesundheit eine Vitamin D-Konzentration von 20 ng/ml für 97,5 % der Bevölkerung völlig auseichend ist. Bislang wurde eine Vitamin D-Konzentration von 30 ng/ml empfohlen. Darüber hinaus wird weiterhin argumentiert, dass "Nahrungsergänzungsmittel und Vitamin D-Zusätze zu Lebensmitteln aus medizinischer Sicht nur dann sinnvoll sind, wenn weitere Risikofaktoren für eine Osteoporose vorliegen - beispielsweise bei älteren Menschen oder bei verminderter Knochendichte". In gleicher Weise warnt auch die Deutsche Gesellschaft für Endokrinologie, dass "eine Überversorgung Gesundheitsrisiken berge und die Einnahme von Vitamin D-Präparaten nur in ärztlich begründeten Fällen notwendig ist", insbesondere vor dem Hintergrund, dass der Organismus ungefähr 80 % des benötigten Vitamin D selbst bildet. Auch ausgehend von den obigen Ausführungen ist die erfindungsgemäß vorgeschlagene gezielte Therapie für die angeführten Erkrankungen auf Basis von Vitamin D dem Fachmann gerade nicht nahegelegt.

Denn erfindungsgemäß wurden nunmehr in völlig überraschender Weise spezifische Wirkeffekte von Vitamin D, insbesondere Calcidiol und/oder Calcitriol, gefunden, welche die erfindungsgemäßen speziellen Verwendungen im Rahmen von Wirkeffizienzsteigerung und Dosisreduktion begleitender Therapeutika bzw. pharmakologischer Wirksubstanzen ermöglichen.

In diesem Zusammenhang wurde überraschenderweise auch gefunden, dass Calcidiol, der Präkursor und Metabolit von Calcitriol, eine primäre und stärker ausgebildete sowie eigenständige steroidartige und entzündungshemmende Wirkung besitzt als Calcitriol bzw. Vitamin D in seiner physiologisch aktiven Form selbst, so dass Calcidiol eine erfindungsgemäß besonders bevorzugte Wirkkomponente in Form des Vitamin D-Rezeptoragonisten (VDA) darstellt.

Insbesondere übersteigt die entzündungshemmende Wirkung von Calcidiol die Wirkung von Calcitriol etwa um das dreifache, so dass Calcidiol, wie zuvor angeführt, im Rahmen des erfindungsgemäßen therapeutischen Einsatzes, insbesondere als Kombinationstherapeutikum zur Wirkungssteigerung von Leitlinientherapeutika, als eine der bevorzugten Substanzen anzuführen ist. Dessen ungeachtet kommt jedoch auch Calcitriol im Hinblick auf die vorliegende Erfindung eine große Rolle als Vitamin D-Rezeptoragonist (VDA) zu.

Für den therapeutischen Einsatz von Calcitriol und/oder Calcidiol bzw. einer Mischung aus beiden vorgenannten Substanzen zur Erhöhung insbesondere lokaler, antientzündlicher Vitamin D-Konzentrationen und somit der Wirksamkeit, wird erfindungsgemäß zur Sicherstellung der optimalen Verfügbarkeit auch ein neuartiges Therapiekonzept zur primären (Lokal-)Therapie, insbesondere in Form eines Nasensprays; zur intranasalen Therapie, insbesondere in Form eines Pulvers und/oder Dosieraerosols; zur Behandlung insbesondere entzündlicher Erkrankungen der oberen Atemwege, wie Rhinitis, bereitgestellt.

Erfindungsgemäß kann das Kombinationstherapeutikum für die Therapie mit Vitamin D in Form einer intranasal applizierbaren Lösung oder Suspension in Form eines Pulvers oder Sprays bereitgestellt werden, insbesondere verbunden mit dem Vorteil, unabhängig vom Körpergewicht und der Lipophilie des Wirkstoffs, auf Basis von Vitamin D eine lokale Schleimhautentzündung, z. B. der Atemwege oder der Nasenschleimhaut, direkt und nicht sekundär über einen mitunter nicht sichergestellten Systemeffekt zu behandeln. Durch diesen Ansatz werden auch gegebenenfalls vorliegende Vitamin D-Nebenwirkungen der oralen Therapie, insbesondere die Hypercalcämie, vermieden. Insofern stellen erfindungsgemäß sowohl der Vitamin D-Rezeptoragonist (VDA) (= *erste lokal, insbesondere nasal applizierbare Komponente)* also auch der Vasokonstriktor (= *zweite lokal, insbesondere nasal applizierbare Komponente*) topisch bzw. lokal applizierbare Komponenten dar, vorzugsweise in einer gemeinsamen Zusammensetzung.

Im Rahmen einer Co-Medikation, beispielsweise mit Kortikosteroiden, kann es erfindungsgemäß in nicht beschränkender Weise zudem vorgesehen sein, dass einerseits der Vitamin D-Rezeptoragonist (VDA), insbesondere gemeinsam mit dem Vasokonstriktor nasal (= *erste und zweite lokal applizierbare Komponente*), beispielsweise in Form eines Nasensprays, verabreicht wird und andererseits - gemeinsam bzw. in Kombination hiermit oder aber zeitlich versetzt hierzu - mindestens ein systemisch applizierbares, insbesondere peroral appliziertes Kortikosteroid (= *systemisch applizierbare Komponente*) insbesondere zu Zwecken der prophylaktischen und/oder therapeutischen Behandlung von insbesondere entzündlichen Erkrankungen der oberen Atemwege, verabreicht wird. Im Allgemeinen kann das Kortikosteroid insbesondere in Form eines lokalen Kortikosteroids, bevorzugt jedoch lokal, insbesondere nasal appliziert werden (= *dritte lokal, insbesondere nasal applizierbare Komonente*):

Gegenstand der vorliegenden Erfindung - gemäß einem ersten Aspekt der vorliegenden Erfindung - ist somit ein Kombinationstherapeutikum, insbesondere pharmazeutisches Kombinationstherapeutikum, zur Verwendung bei der prophylaktischen und/oder therapeutischen topischen Behandlung von entzündlichen Erkrankungen der Nase und/oder der Nasennebenhöhlen, ausgewählt aus Rhinitis, Rhinosinusitis und Sinusitis, wobei das Kombinationstherapeutikum intranasal applizierbar ist und/oder appliziert wird und wobei das Kombinationstherapeutikum - in jeweils pharmazeutisch wirksamen Mengen - umfasst: (a) mindestens einen Vitamin D-Rezeptoragonisten (VDA) in einer Menge im Bereich von 0,0005 Gew.-% bis 1,5 Gew.-%, bezogen auf das Kombinationstherapeutikum, und (b) mindestens einen Vasokonstriktor in Form eines alpha-Sympathomimetikums, vorzugsweise alpha-1-Sympathomimetikums, oder dessen physiologisch unbedenkliche Salze in einer Menge im Bereich von 0,001 Gew.-% bis 2 Gew.-%, bezogen auf das Kombinationstherapeutikum.

Die Begriffe "Kombinationstherapeutikum" bzw. "pharmazeutisches Kombinationstherapeutikum" oder dergleichen, wie sie im Rahmen der vorliegenden Erfindung verwendet werden, sind sehr umfassend zu verstehen und bezeichnen nicht nur pharmazeutische Präparate bzw. Pharmazeutika, sondern auch sogenannte Medizinprodukte, homöopathische Mittel, Nahrungsergänzungsmittel oder dergleichen.

Weiterhin werden unter den Begriffen "Vitamin D-Rezeptoragonist" bzw. "Vitamin D-Rezeptoragonist (VDA)", wie sie im Rahmen der vorliegenden Erfindung verwendet werden, insbesondere solche Substanzen verstanden, welche imstande sind, mit den zugrundeliegenden Vitamin D-Rezeptoren insbesondere nach Art eines Liganden in Wechselwirkung zu treten bzw. mit den zugrundeliegenden Vitamin D-Rezeptoren zu interagieren, so dass infolge der zugrundeliegenden Wechselwirkung bzw. Interaktion eine Aktivierung der in Rede stehenden Vitamin D-Rezeptoren erfolgt. Insbesondere kann die Wechselwirkung bzw. Interaktion des erfindungsgemäß eingesetzten Vitamin D-Rezeptoragonisten mit den Vitamin D-Rezeptoren auf Basis einer Bindung, insbesondere auf Basis des sogenannten "Schlüssel/Schloss-Prinzips" erfolgen, infolgedessen eine Aktivierung der zugrundeliegenden Vitamin D-Rezeptoren mit nachfolgender Induktion stoffwechselspezifischer Vorgänge erfolgen kann. Hierbei kann es insbesondere vorgesehen sein, dass die erfindungsgemäß eingesetzte Substanz als solche, d. h. ohne weiterführende Modifizierung bzw. Verstoffwechselung, mit den Vitamin D-Rezeptoren zu interagieren imstande ist. Gleichermaßen umfassen die Begriffe "Vitamin D-Rezeptoragonist" bzw. "Vitamin D-Rezeptoragonist (VDA)", auch solche Substanzen, welche selbst nicht unmittelbar zur Wechselwirkung, insbesondere Bindung, mit bzw. an den Vitamin D-Rezeptor imstande sind, sondern gewissermaßen als Vorläufersubstanzen bzw. Präkursoren zur Bereitstellung eines Liganden für den Vitamin D-Rezeptor fungieren. Dabei kann die Modifizierung bzw. Umwandlung der eingesetzten Vorläufer insbesondere auf Basis stoffwechselspezifischer Vorgänge im Körper erfolgen.

Im Rahmen der vorliegenden Erfindung können als Vitamin D-Rezeptoragonisten insbesondere den natürlichen bzw. körpereigenen Metaboliten des körpereigenen Vitamin D-Stoffwechsels entsprechende bzw. hierzu chemisch zumindest im Wesentlichen identische bzw. wirkidentische Substanzen auf Basis synthetischer Verbindungen eingesetzt werden, wobei es sich diesbezüglich, wie nachfolgend noch definiert, insbesondere um 25-Hydroxy-Vitamin D₃ bzw. 25-Hydroxy-Vitamin D (Calcidiol) und/oder 1,25-Dihydroxy-Vitamin D₃ bzw. 1,25-Dihydroxy-Vitamin D (Calcitriol) handelt. In diesem Zusammenhang ist es im Rahmen der vorliegenden Erfindung gleichermaßen in nicht beschränkender Weise möglich, die entsprechenden Vorläufersubstanzen bzw. Präkursoren bzw. Speicherformen der in Rede stehenden Substanzen, wie beispielsweise 7-Dehydrocholesterol, Vitamin D₃ (Cholecalciferol) bzw. entsprechende inaktive Speicherprodukte, wie Lumisterol bzw. Tachysterol, einzusetzen, auch wenn dies erfindungsgemäß weniger bevorzugt ist. Insbesondere kann der Vitamin D-Rezeptoragonist auch in Form von Vitamin D₃ bzw. Cholecalciferol als solchem eingesetzt werden, welches nach entsprechender Verabreichung bzw. Applikation - ohne sich auf diese Theorie beschränken zu wollen - im Körper zu den entsprechend aktiven Liganden, insbesondere Calcidiol und/oder Calcitriol, metabolisiert bzw. verstoffwechselt wird.

Im Rahmen der vorliegenden Erfindung ist es, wie zuvor ausgeführt, gleichermaßen möglich, synthetische Analoga von Vitamin D₃ sowie dessen Vorläufern bzw. Präkursoren als Vitamin D-Rezeptoragonisten einzusetzen. Insbesondere kommt im Rahmen der vorliegenden Erfindung auch die Verwendung von Vitamin D₃-Analoga, wie Doxercalciferol, Alphacalciferol, Calcipotriol bzw. Dihydrotachystyrol, als Vitamin D-Rezeptoragonist in Betracht.

Gleichermaßen kommt im Rahmen der vorliegenden Erfindung auch die Verwendung von Vitamin D₂ bzw. Ergocalciferol sowie dessen Analoga in Betracht.

Bei den erfindungsgemäß eingesetzten Vitamin D-Rezeptoragonisten handelt es sich insbesondere um Substanzen, welche in Form von sogenannten Secosteroiden vorliegen können.

Weiterhin beziehen sich die im Rahmen der vorliegenden Erfindung verwendeten Begriffe "Vitamin D-Rezeptor" bzw. "Vitamin D-Rezeptor (VDR)" auf insbesondere zur Familie der Steroidrezeptoren vom Typ II gehörenden ligandenaktivierte Transkriptionsfaktoren bzw. Rezeptoren, welche insbesondere eine Affinität zu Liganden aus der Vitamin D-Gruppe aufweisen, wobei die in Rede stehenden Rezeptoren insbesondere eine hohe (Bindungs-)Affinität zu 25-Hydroxy-Vitamin D (Calcidiol) und/oder 1,25-Dihydroxy-Vitamin D (Calcitriol) aufweisen. Die in Rede stehenden Rezeptoren, welche erfindungsgemäß sozusagen als Zielstruktur bzw. *Target* für die eingesetzten Liganden fungieren, stellen somit im Allgemeinen insbesondere zu der Familie der Steroidrezeptoren gehörende Rezeptoren in Form von ligandenaktivierten Transkriptionsfaktoren dar, die infolge ihrer Aktivierung bestimmte Zielgene zu aktivieren oder zu hemmen im Stande sind, und so den Stoffwechsel insbesondere auch im Hinblick auf die Steuerung entzündlicher Prozesse beeinflussen können.

Ausgehend von der hohen Bindungsaffinität des Vitamin D-Rezeptors zu Calcidiol bzw. Calcitritol resultiert auch dessen Funktion, nämlich die Vermittlung der physiologischen Funktionen von Vitamin D in den zugrundeliegenden biologischen Strukturen, insbesondere Zielzellen. Ohne sich auf diese Theorie beschränken zu wollen, bilden Vitamin D-Rezeptoren nach Aktivierung durch Anbindung des Liganden ein Heterodimer mit dem sogenannten Retinoid-X-Rezeptor, wobei das resultierende Heterodimer als regulatives Element für die Expression von Zielgenen des Vitamin D-Rezeptors dient. Insbesondere kommt Vitamin D-Rezeptoren eine wichtige Funktion in der Regulation von Genen bzw. Genprodukten zu, welche für Immunantworten, insbesondere entzündliche Prozesse unter Prostaglandinausschüttung sowie für proliferative Prozesse von Bedeutung sind.

Im Rahmen der vorliegenden Erfindung kann es vorgesehen sein, dass (a) der Vitamin D-Rezeptoragonist (VDA) ausgewählt ist aus der Gruppe von Verbindungen der Vitamin D-Gruppe, insbesondere Vitamin D₃ (Cholecalciferol) und/oder Vitamin D₂ (Ergocaliferol), vorzugsweise Vitamin D₃ (Cholecalciferol).

Gleichermaßen kann es im Rahmen der vorliegenden Erfindung vorgesehen sein, dass (a) der Vitamin D-Rezeptoragonist (VDA) ausgewählt ist aus der Gruppe von insbesondere physiologischen Vitamin D₃-Metaboliten und/oder insbesondere physiologischen Vitamin D₃-Präkursoren, vorzugsweise aus der Gruppe von insbesondere physiologischen Vitamin D₃-Metaboliten. Insbesondere kann (a) der Vitamin D-Rezeptoragonist (VDA) ausgewählt sein aus der Gruppe von insbesondere synthetischen Vitamin D₃-Analoga, insbesondere Tacalcitol (1,24-Dihydroxy-Vitamin D₃).

Erfindungsgemäß werden im Hinblick auf die Behandlung von insbesondere entzündlichen Erkrankungen der oberen Atemwege besonders gute Ergebnisse erhalten, wenn der Vitamin D-Rezeptoragonist (VDA) ausgewählt ist aus der Gruppe von 25-Hydroxy-Vitamin D₃ (Calcidiol) und/oder 1,25-Dihydroxy-Vitamin D₃ (Calcitriol), vorzugsweise 25-Hydroxy-Vitamin D₃ (Calcidiol). Gemäß einer erfindungsgemäß besonders bevorzugten Ausführungsform ist (a) der Vitamin D-Rezeptoragonist (VDA) die Substanz 25-Hydroxy-Vitamin D₃ (Calcidiol).

Bei 25-Hydroxy-Vitamin D bzw. Calcidiol, welches synonym auch als 25-OH-Vitamin D₃ bzw. 25-OH D₃ bzw. 25-Hydroxycholecalciferol bezeichnet wird, handelt es sich insbesondere um eine Substanz des Vitamin D-Stoffwechsels mit Ligandenfunktion in Bezug auf Vitamin D-Rezeptoren. Im menschlichen Körper kann Calcidiol ausgehend von Vitamin D₃ durch Hydroxylierung von Vitamin D₃ insbesondere in der Leber gebildet werden.

Weiterhin handelt es sich bei 1,25-Dihydroxy-Vitamin D₃ bzw. 1,25-Dihydroxyvitamin D bzw. Calcitriol, synonym auch als 1,25-(OH)₂-Vitamin D₃, 1.25-DOH D₃ bzw. 1-α-25 (OH)₂-Cholecalciferol bezeichnet, um eine weitere Substanz des Vitamin D₃-Stoffwechsels, wobei Calcitriol aus weiterführender Hydroxylierung von Calcidiol resultieren kann.

Erfindungsgemäß kann es vorgesehen sein, dass (a) der Vitamin D-Rezeptoragonist (VDA) eine Kombination von 25-Hydroxy-Vitamin D₃ (Calcidiol) und 1,25-Dihydroxy-Vitamin D₃ (Calcitriol) ist.

In diesem Zusammenhang hat es sich als besonders vorteilhaft erwiesen, wenn das Kombinationstherapeutikum (i) 25-Hydroxy-Vitamin D₃ und (ii) 1,25-Dihydroxy-Vitamin D₃ in einem gewichtsbezogenen Mengenverhältnis von [(i) : (ii)] im Bereich von 1.000 : 1 bis 1 : 100, insbesondere im Bereich von 500 : 1 bis 1 : 10, vorzugsweise im Bereich von 100 : 1 bis 1 : 5, bevorzugt im Bereich von 50 : 1 bis 1 : 1, besonders bevorzugt im Bereich von 10 : 1 bis 1 : 1, aufweist.

Gemäß einer erfindungsgemäß weiter bevorzugten Ausführungsform kann der Vitamin D-Rezeptoragonist (VDA) 25-Hydroxy-Vitamin D₃ (Calcidiol) sein.

Im Allgemeinen kann die Menge an Vitamin D-Rezeptoragonist (VDA) in dem erfindungsgemäßen Kombinationstherapeutikum in weiten Bereichen variieren. Im Rahmen der vorliegenden Erfindung ist es vorgesehen, insbesondere um eine ausreichende Wirksamkeit zu gewährleisten, dass das Kombinationstherapeutikum (a) den Vitamin D-Rezeptoragonisten (VDA), insbesondere 25-Hydroxy-Vitamin D₃ (Calcidiol) und/oder 1,25-Dihydroxy-Vitamin D₃ (Calcitriol), in einer Menge im Bereich von 0,0005 Gew.-% bis 1,5 Gew.-%, insbesondere im Bereich von 0,001 Gew.-% bis 1,25 Gew.-%, vorzugsweise im Bereich von 0,005 Gew.-% bis 1 Gew.-%, bevorzugt im Bereich von 0,01 Gew.-% bis 0,5 Gew.-%, besonders bevorzugt im Bereich von 0,02 Gew.-% bis 0,3 Gew.-%, ganz besonders bevorzugt im Bereich von 0,03 Gew.-% bis 0,15 Gew.-%, bezogen auf das Kombinationstherapeutikum, enthält.

Was das erfindungsgemäße Kombinationstherapeutikum weiterhin anbelangt, so ist es erfindungsgemäß bevorzugt, wenn (b) der Vasokonstriktor ein imidazolinbasiertes alpha-Sympathomimetikum ist.

In diesem Zusammenhang kann es erfindungsgemäß vorgesehen sein, dass (b) der Vasokonstriktor ausgewählt ist aus der Gruppe von Xylometazolin, Oxymetazolin, Naphazolin, Tetryzolin, Tramazolin, Phenylephrin und deren physiologisch unbedenklichen Salzen, insbesondere in Form von deren Hydrochloridsalzen, sowie Mischungen und Kombinationen der vorgenannten Verbindungen; vorzugsweise Xylometazolin, Oxymetazolin und deren physiologisch unbedenklichen Salzen, insbesondere in Form von deren Hydrochloridsalzen.

Erfindungsgemäß ist es in diesem Zusammenhang bevorzugt, wenn (b) der Vasokonstriktor ausgewählt ist aus der Gruppe von Xylometazolin und Oxymetazolin, insbesondere in Form von deren physiologisch unbedenklichen Salzen, besonders bevorzugt in Form von deren Hydrochloridsalzen.

Gemäß einer besonders bevorzugten Ausführungsform sollte (b) der Vasokonstriktor Xylometazolin sein, insbesondere in Form von dessen physiologisch unbedenklichen Salzen, besonders bevorzugt in Form von dessen Hydrochloridsalz (Xylometazolinhydrochlorid).

Die Menge an Komponente (b) in dem erfindungsgemäßen Kombinationstherapeutikum kann gleichermaßen in weiten Bereichen variieren.

Um eine ausreichende Wirkung der Komponente (b) in dem erfindungsgemäßen Kombinationstherapeutikum zu gewährleisten, ist es erfindungsgemäß vorgesehen, dass das Kombinationstherapeutikum den (b) Vasokonstriktor in einer Menge im Bereich von 0,001 Gew.-% bis 2 Gew.-%, insbesondere im Bereich von 0,005 Gew.-% bis 1,5 Gew.-%, vorzugsweise im Bereich von 0,01 Gew.-% bis 1,2 Gew.-%, bevorzugt im Bereich von 0,02 Gew.-% bis 1,0 Gew.-%, besonders bevorzugt im Bereich von 0,03 Gew.-% bis 0,5 Gew.-%, ganz besonders bevorzugt im Bereich von 0,04 Gew.-% bis 0,2 Gew.-%, bezogen auf das Kombinationstherapeutikum, enthält.

Aufgrund des Vorhandenenseins eines Vasokonstriktors wird bei intranasaler Applikation bzw. Anwendung des erfindungsgemäßen Kombinationstherapeutikums, insbesondere bei der Behandlung von Rhinitiden, aufgrund der vasokonstriktorischen Eigenschaften der Wirkkomponente (b) eine effektive Abschwellung der Nasenschleimhäute, einhergehend mit einer besseren Belüftung auch der Nasennebenhöhlen, erreicht, wobei sich die diesbezügliche Wirkung durch die gezielte Kombination mit dem Vitamin D-Rezeptoragonisten (VDA) in synergistischer Weise verstärkt, was im Ergebnis zu einer hohen Wirkeffizienz des erfindungsgemäßen Kombinationstherapeutikums in Bezug auf die angeführten Erkrankungen mit den diesbezüglichen Indikationen führt.

In diesem Zusammenhang kommt auch dem mengenspezifischen Verhältnis von (a) Vitamin D-Rezeptoragonist (VDA) einerseits und (b) Vasokonstriktor andererseits in dem erfindungsgemäßen Kombinationstherapeutikum eine große Bedeutung zu. In diesem Zusammenhang werden hinsichtlich der Wirkeffizienz des erfindungsgemäßen Kombinationstherapeutikums besonders gute Ergebnisse erhalten, wenn das Kombinationstherapeutikum (a) den Vitamin D-Rezeptoragonisten (VDA) und (b) den Vasokonstriktor in einem gewichtsbezogenen Mengenverhältnis von [(a) : (b)] im Bereich von 1 : 100 bis 10 : 1, insbesondere im Bereich von 1 : 50 bis 5 : 1, vorzugsweise im Bereich von 1 : 10 bis 2 : 1, bevorzugt im Bereich von 1 : 5 bis 1 : 1, besonders bevorzugt im Bereich von 1 : 3 bis 1 : 1,5, enthält.

Erfindungsgemäß kann es zudem vorgesehen sein, dass das Kombinationstherapeutikum (c) Pantothenol (Dexpanthenol) oder dessen physiologisch unbedenkliche Ester und/oder Pantothensäure oder deren physiologisch unbedenkliche Salze, vorzugsweise Pantothenol (Dexpanthenol) oder dessen physiologisch unbedenkliche Ester, bevorzugt Pantothenol (Dexpanthenol), enthält.

In diesem Zusammenhang sollte - um eine ausreichende Wirkung durch die Wirkkomponente (c) zu erhalten - das Kombinationstherapeutikum (c) Pantothenol (Dexpanthenol) oder dessen physiologisch unbedenkliche Ester und/oder Pantothensäure oder deren physiologisch unbedenkliche Salze in einer Menge im Bereich von 0,01 Gew.-% bis 20 Gew.-%, insbesondere im Bereich von 0,1 Gew.-% bis 15 Gew.-%, vorzugsweise im Bereich von 0,5 Gew.-% bis 10 Gew.-%, bevorzugt im Bereich von 1 Gew.-% bis 7 Gew.-%, besonders bevorzugt im Bereich von 2 Gew.-% bis 6 Gew.-%, ganz besonders bevorzugt im Bereich von 3 Gew.-% bis 6 Gew.-%, bezogen auf das Kombinationstherapeutikum, enthalten.

Durch den gezielten Einsatz der Wirkkomponente (c) auf Basis von Dexpanthenol bzw. dessen physiologisch unbedenklichen Estern bzw. Pantothensäure oder deren physiologisch unbedenklichen Salzen wird einer Austrocknung insbesondere der Nasenschleimhäute und entzündlichen Irritationen, insbesondere infolge der Anwendung des alpha-Sympathomimetikum, noch weiter entgegengewirkt.

Erfindungsgemäß kann es vorgesehen sein, dass das Kombinationstherapeutikum (d) mindestens ein vorzugsweise saures Glykosaminoglykan oder dessen physiologisch unbedenkliche Salze, insbesondere Hyaluronsäure oder deren Salze, vorzugsweise das Natriumsalz der Hyaluronsäure, enthält.

Um eine ausreichende Wirkung durch die Wirkkomponente (d) sicherzustellen, sollte in diesem Zusammenhang das Kombinationstherapeutikum (d) das mindestens eine vorzugsweise saure Glykosaminoglykan oder dessen physiologisch unbedenkliche Salze in einer Menge im Bereich von 0,05 Gew.-% bis 3 Gew.-%, vorzugsweise im Bereich von 0,2 Gew.-% bis 2 Gew.-%, bevorzugt im Bereich von 0,1 Gew.-% bis 1 Gew.-%, besonders bevorzugt im Bereich von 0,05 Gew.-% bis 0,5 Gew.-%, ganz besonders bevorzugt im Bereich von 0,05 Gew.-% bis 0,25 Gew.-%, bezogen auf das Kombinationstherapeutikum, enthalten.

Weiterhin kann es erfindungsgemäß zudem vorgesehen sein, dass das Kombinationstherapeutikum (e) Ectoin oder mindestens ein Ectoinderivat, insbesondere ein Hydroxyectoin, enthält. In diesem Zusammenhang liegt die Kompnente (e) insbesondere in einer Menge im Bereich von 0,0001 Gew.-% bis 10 Gew.-%, insbesondere im Bereich von 0,001 Gew.-% bis 5 Gew.-%, vorzugsweise im Bereich von 0,01 Gew.-% bis 2 Gew.-%, bezogen auf das Kombinationstherapeutikum, vor.

Zudem kann es erfindungsgemäß vorgesehen sein, dass das Kombinationstherapeutikum mindestens einen weiteren pharmakologisch wirksamen Inhaltsstoff bzw. Wirkstoff enthält. Auf diese Weise kann die Wirksamkeit des erfindungsgemäßen Kombinationstherapeutikums (f) insbesondere im Hinblick auf die jeweilige Indikation optimiert bzw. maßgeschneidert werden.

In diesem Zusammenhang kann (f) der weitere pharmakologisch wirksame Inhaltsstoff und/oder Wirkstoff ein Antiallergikum, insbesondere Antihistaminikum und/oder Mastzellstabilisator, sein. Diesbezüglich kann das Antiallergikum ausgewählt sein aus der Gruppe von Azelastin, Levocabstin, Cromoglycinsäure und Nedocromil sowie Mischungen und Kombinationen der vorgenannten Verbindungen.

Ein derartiger pharmakologisch wirksamer Inhalts- bzw. Wirkstoff kommt insbesondere bei der Behandlung von *Rhinitis allergica* bzw. Heuschnupfen in Betracht.

Gleichermaßen kann der (f) weitere pharmakologisch wirksame Inhaltsstoff und/oder Wirkstoff in nichtbeschränkender Weise ein insbesondere topisch applizierbares, vorzugsweise nasal applizierbares Kortikosteroid, insbesondere Glukokortikosteroid, sein. In diesem Zusammenhang kann das Glukokortikosteroid ausgewählt sein aus der Gruppe von Dexamethason, Budesonid, Prednison, Prednisolon, Betamethason, Rimexolon und Fluorometholon sowie Mischungen und Kombinationen der vorgenannten Verbindungen.

Auch die vorgenannten Kortikosteroide kommen beispielsweise bei der Behandlung von *Rhinitis allergica* oder dergleichen in Betracht.

Um eine entsprechende Wirksamkeit der Wirkkomponente (f) zu gewährleisten, sollte das Kombinationstherapeutikum den (f) weiteren pharmakologisch wirksamen Inhaltsstoff und/oder Wirkstoff in einer Menge im Bereich von 0,001 Gew.-% bis 5 Gew.-%, insbesondere im Bereich von 0,01 Gew.-% bis 2 Gew.-%, vorzugsweise im Bereich von 0,1 Gew.-% bis 1 Gew.-%, bezogen auf das Kombinationstherapeutikum, enthalten.

Im Allgemeinen kann das Kombinationstherapeutikum als insbesondere wässrige Zusammensetzung vorliegen bzw. kann das Kombinationstherapeutikum wässrig basiert sein und/oder wässrig formuliert vorliegen, insbesondere in Form einer wässrigen Lösung oder wässrigen Solubilisierung und/oder Emulsion. Gleichermaßen kann es erfindungsgemäß aber auch möglich sein, dass das Kombinationstherapeutikum nach der Erfindung als Trockenformulierung, insbesondere als Pulver, vorzugsweise Sprühpulver, vorliegt.

Weiterhin kann es erfindungsgemäß vorgesehen sein, dass das Kombinationstherapeutikum insbesondere im Fall der wässrigen Formulierung einen pH-Wert im Bereich von 5,0 bis 7,0, insbesondere im Bereich von 5,1 bis 6,5, vorzugsweise im Bereich von 5,2 bis 6,0, aufweist. Hierdurch kann zum einen die Stabilität der Zusammensetzung, insbesondere im Hinblick auf den Vasokonstriktor, erhöht und zudem die Löslichkeit von Vitamin D bzw. des Vitamin D-Rezeptoragonisten (VDA) verbessert werden.

Im Rahmen der vorliegenden Erfindung kommt es zudem in Betracht, dass das Kombinationstherapeutikum mindestens einen weiteren Inhaltsstoff aufweist. In diesem Zusammenhang sollte der weitere Inhaltsstoff ausgewählt sein aus der Gruppe von Verarbeitungshilfsstoffen, Stabilisatoren, insbesondere organischen Lösemitteln, Emulgatoren, Antioxidantien, Feuchthaltemitteln, Verdickungsmitteln, Antiseptika, Farbstoffen, Aromastoffen, Riechstoffen, Duftstoffen, Streckmitteln, Bindemitteln, Netzmitteln, Vitaminen, Spurenelementen, Mineralstoffen, Mikronährstoffen und/oder ätherischen Ölen sowie deren Kombinationen.

Weiterhin kann es im Rahmen der vorliegenden Erfindung vorgesehen sein, dass das Kombinationstherapeutikum den Vitamin D-Rezeptoragonisten (VDA) zusammen mit mindestens einem mit dem Vitamin D-Rezeptoragonisten (VDA) mischbaren und/oder hierin löslichen, insbesondere bei 20 °C und Atmosphärendruck flüssigen, physiologisch unbedenklichen Träger (Exzipienten) enthält. Der Träger kann dabei ausgewählt sein aus der Gruppe von fetten Ölen, bevorzugt Triglyceriden, besonders bevorzugt mittelkettigen Triglyceriden *(Medium Chain Triglycerides,* MCT), ganz besonders bevorzugt Triglyceriden mit C₆-C₁₂-Fettsäureresten. Gleichermaßen kann der Träger in einem Vitamin D-Rezeptoragonist (VDA)/Träger-Mengenverhältnis im Bereich von 5 : 1 bis 1 : 100, insbesondere 1 : 1 bis 1 : 10, eingesetzt sein. Das Kombinationstherapeutikum nach der Erfindung kann zudem mindestens einen Emulgator enthalten, insbesondere wobei der Emulgator ausgewählt ist aus der Gruppe von Polyvinylalkoholen, Polyethylenglykolen, Propylenglykolen und Polyethylenglykolethylestern sowie Mischungen und Kombinationen der vorgenannten Verbindungen.

Der Begriff der Öle, wie er erfindungsgemäß für die vorstehenden Träger bzw. Exzipienten verwendet wird, ist eine Sammelbezeichnung für Flüssigkeiten, welche sich nicht mit Wasser mischen lassen. Der Begriff der fetten Öle, wie er in diesem Zusammenhang erfindungsgemäß verwendet wird, bezeichnet speziell Fette, d. h. Gemische von Fettsäuretriglyceriden, welche bei Raumtemperatur (insbesondere 20 °C) und Atmosphärendruck flüssig sind; insbesondere bezeichnet dieser Begriff Ester des dreiwertigen Alkohols Glycerin (Propan-1,2-3-triol) mit drei, meist verschiedenen, überwiegend geradzahligen und unverzweigten aliphatischen Monocarbonsäuren, den so genannten Fettsäuren. Verbindungen dieser Art werden auch Triglyceride genannt (nach IUPAC-Empfehlung: Triacylglycerine). Triglyceride, synonym auch als Glycerol-Triester bezeichnet, sind also dreifache Ester des dreiwertigen Alkohols Glycerin mit drei Säuremolekülen, wobei die Vorsilbe *"tri"* auf drei Acyl-Säurereste, die mit Glycerin verestert sind, verweist.

Speziell mittelkettige Triglyceride, wie sie erfindungsgemäß als Träger bzw. Exzipient zum Einsatz kommen können, sind insbesondere halbsynthetische neutrale Glycerinester von gesättigten, im Allgemeinen unverzweigten Monocarbonsäuren mittlerer Kettenlänge (d. h. C₆-C₁₂). Insbesondere bezeichnet der Begriff der mittelkettigen Triglyceride Gemische von Triglyceriden gesättigter Fettsäuren, hauptsächlich Caprylsäure (Octansäure) und Caprinsäure (Decansäure). Mittelkettige Triglyceride können im Allgemeinen aus Öl hergestellt werden, das aus dem festen und getrockneten Teil des Endosperms von *Cocos nucifera* L. und/oder aus dem getrockneten Endosperm von *Elaeis guineenses* Jacq. extrahiert wird. Für weitergehende Einzelheiten zu dem Begriff der mittelkettigen Triglyceride kann beispielsweise verwiesen werden auf die Monographie Ph. Eur. 6., Ausgabe, Grundwerk 2008, Seiten 4224 bis 4226, sowie auf die Zeitschrift für Ernährungswissenschaft, Band 13, Heft 1/2, 1973, Seiten 6 ff., D. Sailer et al. "Mittelkettige Triglyceride - Klinische Physiologie und Anwendung*"*)*.*

Auf dieser Basis kann das erfindungsgemäße Kombinationstherapeutikum in Form einer wässrig basierten Emulsion vorliegen, insbesondere in Form einer Öl-in-Wasser-Emulsion.

Im Rahmen der vorliegenden Erfindung ist es jedoch gleichermaßen möglich, eine Derivatisierung des Vitamin D-Rezeptoragonisten (VDA) durchzuführen, um auf diese Weise das Löslichkeitsverhalten, insbesondere die Hydrophilie, des Vitamin D-Rezeptoragonisten (VDA) insbesondere gegenüber dem dem Kombinationstherapeutikum zugrundeliegenden Lösemittel, insbesondere Wasser, zu erhöhen.

In erfindungsgemäßer Weise ist es vorgesehen, dass das Kombinationstherapeutikum nach der Erfindung intranasal applizierbar ist und/oder appliziert ist bzw. appliziert wird.

In diesem Zusammenhang kann das Kombinationstherapeutikum in Tagesdosen von 1 µg bis 1.000 µg, insbesondere 10 µg bis 800 µg, vorzugsweise 20 µg bis 500 µg, Vitamin D-Rezeptoragonist (VDA) verabreicht werden. Insbesondere kann das Kombinationstherapeutikum zur Verabreichung in einer Tagesdosis von 1 µg bis 1.000 µg, insbesondere 10 µg bis 800 µg, vorzugsweise 20 µg bis 500 µg, Vitamin D-Rezeptoragonist (VDA) hergerichtet sein.

Weiterhin betrifft die vorliegenden Erfindung - gemäß einem weiteren Aspekt der vorliegenden Erfindung - ein Kombinationstherapeutikum, insbesondere wie zuvor definiert, in Form eines Kits zur Verwendung bei der prophylaktischen und/oder therapeutischen topischen Behandlung von entzündlichen Erkrankungen der Nase und/oder der Nasennebenhöhlen, ausgewählt aus Rhinitis, Rhinosinusitis und Sinusitis, umfassend - in jeweils pharmazeutisch wirksamen Mengen - mindestens einen Vitamin D-Rezeptoragonisten (VDA) in einer Menge im Bereich von 0,0005 Gew.-% bis 1,5 Gew.-%, bezogen auf das Kombinationstherapeutikum, einerseits und mindestens einen Vasokonstriktor in Form eines alpha-Sympathomimetikums, vorzugsweise alpha-1-Sympathomimetikums, oder dessen physiologisch unbedenkliche Salze in einer Menge im Bereich von 0,001 Gew.-% bis 2 Gew.-%, bezogen auf das Kombinationstherapeutikum, andererseits, wobei das Kombinationstherapeutikum intranasal applizierbar ist und/oder appliziert wird.

Gleichermaßen eignet sich das Kombinationstherapeutikum, wie zuvor definiert, zur Verwendung zur prophylaktischen und/oder therapeutischen topischen Behandlung von entzündlichen Erkrankungen der der Nase und/oder der Nasennebenhöhlen, ausgewählt aus Rhinitis, Rhinosinusitis und Sinusitis, bzw. zur Verwendung zur Herstellung eines Arzneimittels zur prophylaktischen und/oder therapeutischen topischen Behandlung von entzündlichen Erkrankungen der Nase und/oder der Nasennebenhöhlen, ausgewählt aus Rhinitis, Rhinosinusitis und Sinusitis, wobei das Kombinationstherapeutikum intranasal applizierbar ist und/oder appliziert wird und wobei das Kombinationstherapeutikum - in jeweils pharmazeutisch wirksamen Mengen - mindestens einen Vitamin D-Rezeptoragonisten (VDA) in einer Menge im Bereich von 0,0005 Gew.-% bis 1,5 Gew.-%, bezogen auf das Kombinationstherapeutikum, einerseits und mindestens einen Vasokonstriktor in Form eines alpha-Sympathomimetikums, vorzugsweise alpha-1-Sympathomimetikums, oder dessen physiologisch unbedenkliche Salze in einer Menge im Bereich von 0,001 Gew.-% bis 2 Gew.-%, bezogen auf das Kombinationstherapeutikum, andererseits umfasst.

Ebenfalls eignet sich der Vitamin D-Rezeptoragonist (VDA) zur Verwendung zur prophylaktischen und/oder therapeutischen Behandlung von insbesondere entzündlichen Erkrankungen der oberen Atemwege, vorzugsweise der Nase und/oder der Nasennebenhöhlen, vorzugsweise Rhinitis, insbesondere wobei der Vitamin D-Rezeptoragonist (VDA) zusammen mit mindestens einem Vasokonstriktor, insbesondere alpha-Sympathomimetikum, vorzugsweise alpha-1-Sympathomimetikum, oder dessen physiologisch unbedenklichen Salzen verabreicht und/oder eingesetzt wird.

Was das erfindungsgemäße Kombinationstherapeutikum weiterhin anbelangt, so eignet sich dieses zur Verwendung bei der prophylaktischen und/oder therapeutischen topischen Behandlung von *Rhinitis acuta,* bzw. zur prophylaktischen und/oder therapeutischen topischen Behandlung von *Rhinitis acuta, Rhinitis allergica, Rhinitis atrophicans, Rhinitis hyperplastica* oder *hypertrophicans, Rhinitis mutilans, Rhinitis nervosa* oder *vasomotorica* oder *Rhinitis pseudomembranacea,* vorzugsweise *Rhinitis acuta* oder *Rhinitis allergica.*

Insbesondere eignet sich das erfindungsgemäße Kombinationstherapeutikum zur Verwendung bei der prophylaktischen und/oder therapeutischen topischen Behandlung von polypoider Rhinitis, insbesondere chronischer und/oder chronisch rezidivierender Rhinitis, und/oder zur prophylaktischen und/oder therapeutischen topischen Behandlung von Sinusitis und/oder Rhinosinusitis.

Darüber hinaus eignet sich das erfindungsgemäße Kombinationstherapeutikum gleichermaßen zur Intensivierung der Wirksamkeit und/oder Wirksamkeitssteigerung und/oder Dosisreduktion von Steroiden bei Erkrankungen der oberen Atemwege, insbesondere der akuten, allergischen, chronischen und/oder chronisch rezidivierenden, polypoiden Rhinitis und/oder Rhinosinusitis, insbesondere wobei das Steroid ein insbesondere topisch applizierbares, vorzugsweise nasal applizierbares Kortikosteroid, insbesondere Glukokortikosteroid, ist, vorzugsweise ausgewählt aus der Gruppe von Dexamethason, Budesonid, Prednison, Prednisolon, Betamethason, Rimexolon und Fluorometholon sowie Mischungen und Kombinationen der vorgenannten Verbindungen. Auch diesbezüglich kann das Kombinationstherapeutikum als flüssige Formulierung, insbesondere Sprühformulierung, vorzugsweise Nasenspray, vorliegen. In diesem Zusammenhang kommt insbesondere eine Co-Medikation des erfindungsgemäßen Kombinationstherapeutikums mit den zuvor angeführten Steroiden in Betracht.

Darüber hinaus eignet sich das erfindungsgemäße Kombinationstherapeutikum gleichermaßen zur Verwendung bei der prophylaktischen und/oder therapeutischen lokalen Behandlung von insbesondere entzündlichen Erkrankungen der Nase, Nasennebenhöhlen und/oder zur Wirkungsvermittlung und/oder Intensivierung der Wirksamkeit und/oder Wirksamkeitssteigerung abschwellender, antiviraler, antibakterieller, antientzündlicher, antipolypoider und/oder antiallergischer Wirkstoffe, insbesondere wobei das Kombinationstherapeutikum als flüssige Formulierung, insbesondere Sprüh- oder Tropfformulierung, vorzugsweise Nasenspray, vorliegt. Auch diesbezüglich kommt eine Co-Medikation in Betracht, beispielsweise mit Antihistaminika, Chromoglicinsäure, Nedocromil sowie Vagolytika.

Insbesondere eignet sich das erfindungsgemäße Kombinationstherapeutikum auch zur Bereitstellung einer entzündungshemmenden Wirkung für den Einsatz bei Rhinitis, allergischer Rhinitis bzw. chronischer Rhinosinusitis, gegebenenfalls in Kombination mit *Polyposis nasi.* Hierzu kann das Kombinationstherapeutikum zudem Dexpanthenol enthalten bzw. als flüssige Formulierung, insbesondere Sprühformulierung, vorzugsweise Nasenspray, vorliegen.

Insbesondere eignet sich das erfindungsgemäße Kombinationstherapeutikum auch zur Verhinderung oder Reduktion von Wirkverlusten durch Gewöhnungseffekte und/oder Previnismus, insbesondere mit anhaltender nasaler Kongestion und/oder mit Flussminderung, wobei das Kombinationstherapeutikum mindestens ein Steroid, vorzugsweise topisch applizierbares, vorzugsweise nasal applizierbares Kortikosteroid, insbesondere Glukokortikosteroid enthalten kann. Auch hierzu das Kombinationstherapeutikum in nicht beschränkender Weise als flüssige Formulierung, insbesondere Sprühformulierung, vorzugsweise Nasenspray, vorliegen.

Das erfindungsgemäße Kombinationstherapeutikum eignet sich gleichermaßen zur Verwendung bei der prophylaktischen und/oder therapeutischen Behandlung von allergischen Erkrankungen, insbesondere der allergischen Rhinitis, insbesondere mit dem Ziel der Intensivierung bzw. Wirkverbesserung bzw. Wirksamkeitssteigerung von Leitlinientherapien zu den vorgenannten Indikationen. Hierzu kann das Kombinationstherapeutikum als Kit vorliegen und/oder in Verbindung bzw. Co-Medikation mit Leukotrienrezeptorantagonisten, insbesondere Montelukast, Pranlukast, Zaforlukast, 5-Dipoxydinase-Hemmstoffen, Antiallergika und/oder Antihistaminika verabreicht bzw. appliziert werden.

Im Rahmen der vorliegenden Erfindung wurde insbesondere und in völlig überraschender Weise eine synergistische Wirkung von Vitamin D-Rezeptoragonisten (VDA) und Vasokonstriktoren, insbesondere alpha-Sympathomimetika, gefunden.

Erfindungsgemäß eignet sich das Kombinationstherapeutikum nach der Erfindung insbesondere zur Dosisreduktion des Vasokonstriktors in Form des alpha-Sympatomimetikums und/oder zur Dosisreduktion mindestens eines weiteren pharmakologischen Wirk- und/oder Inhaltsstoffs, insbesondere wie zuvor definiert, vorzugsweise eines Kortikosteroids. Insbesondere können die zu verabreichenden Mengen und/oder Dosen des Vasokonstrikors und/oder des weiteren pharmakologischen Wirk- und/oder Inhaltsstoffs, unabhängig voneinander und insbesondere nach einem definierten Anwendungszeitraum des erfindungsgemäßen Kombinationstherapeutikums, z. B. nach drei Tagen, um mindestens 5 %, insbesondere mindestens 10 %, vorzugsweise mindestens 20 %, bevorzugt mindestens 40 %, bezogen auf die ein entsprechendes Therapeutikum in einem Therapieansatz ohne Vitamin D-Rezeptoragonist (VDA), verringert werden.

Weiterer Gegenstand der vorliegenden Erfindung ist zudem die Applikationsvorrichtung nach der Erfindung für die intranasale Applikation, bevorzugt in Form eines Behältnisses mit Tropf- oder Sprüheinrichtung, enthaltend ein Kombinationstherapeutikum, wie es zuvor beschrieben wurde.

In diesem Zusammenhang kann die Applikationsvorrichtung nach der Erfindung eine Sprüheinrichtung zur insbesondere gleichförmigen Ausbringung der Zusammensetzung in einer Menge pro Sprühstoß im Bereich von 20 µl bis 300 µl, insbesondere im Bereich von 30 µl bis 200 µl, vorzugsweise im Bereich von 40 µl bis 100 µl, aufweisen.

Zudem kann die Applikationsvorrichtung nach der Erfindung eine Tropfeinrichtung zur gleichförmigen Ausbringung der Zusammensetzung in einer Menge pro Tropfen im Bereich von 10 µl bis 200 µl, insbesondere im Bereich von 20 µl bis 150 µl, vorzugsweise im Bereich von 15 µl bis 100 µl, aufweisen.

Schließlich kann die Applikationsvorrichtung nach der Erfindung einen Vorratsbehälter mit einem Volumen im Bereich von 0,1 ml bis 100 ml, insbesondere im Bereich von 1 ml bis 50 ml, vorzugsweise im Bereich von 0,1 ml bis 100 ml, aufweisen.

Im Rahmen der erfindungsgemäßen Konzeption kann Vitamin D, insbesondere Calcidiol und/oder Calcitriol, vorzugsweise Calcidiol, als neue Medikation in Form eines zuvor definierten Kombinationstherapeutikums eingesetzt werden, insbesondere auch in Form einer Co-Medikation zusammen mit einer Leitlinientherapie in verschiedenen Kombinationen, insbesondere mit dem Ziel, die eigenständige antiinflammatorische Wirkung von Vitamin D in Kombination mit verschiedenen Leitlinientherapeutika auch unabhängig von einem Vitamin D-Mangel beispielsweise zur Verstärkung der Steroidwirkung von Leitlinientherapeutika einzusetzen, z. B. bei allergischem Schnupfen. Insbesondere kann auf dieser Basis auch der Bedarf an Steroiden gesenkt werden bzw. kann eine Alternative zur Therapie mit Steroiden bereitgestellt werden.

Erfindungsgemäß können zudem insbesondere noch weitere Behandlungskonzepte auf Basis einer Erzielung einer antibiotischen und/oder entzündungshemmmenden Wirkung durch eine Kombination von Vitamin D bzw. Vitamin D-Rezeptoragonist und Vasokonstriktor mit Antibiotika und gegebenenfalls Kortikosteroiden bereitgestellt werden.

Weitere Ausgestaltungen, Abwandlungen und Variationen sowie Vorteile der vorliegenden Erfindung sind für den Fachmann beim Lesen der Beschreibung ohne Weiteres erkennbar und realisierbar, ohne dass er dabei den Rahmen der vorliegenden Erfindung verlässt.

Die folgenden Ausführungsbeispiele dienen lediglich der Veranschaulichung der vorliegenden Erfindung, ohne sie jedoch hierauf zu beschränken.

### Ausführungsbeispiele und weitere Ausführungen zur vorliegenden Erfindung:

### Methodik

Im Rahmen der vorliegenden Erfindung wurde der Einfluss der entzündungshemmenden Wirkung von Calcidiol und Calcitriol sowie gegebenenfalls von verschiedenen weiteren Testsubstanzen in Kulturen von normalen humanen Monozyten *in vitro* untersucht. Als Maß zur Bestimmung der entzündungshemmenden Wirkung wurde die Stärke der Hemmung der Produktion des Tumornekrosefaktor-alpha (TNF-alpha), repräsentativ für die mit entzündlichen Prozessen assoziierte Zytokinproduktion, in humanen Monozyten bestimmt. Die Produktion von TNF-alpha kann in humanen Monozyten gezielt durch die Zugabe von Lipopolysacchariden (LPS) stimuliert werden. Je stärker die entzündungshemmende Wirkung einer Testsubstanz ist, desto weniger TNF-alpha wird durch die mit LPS stimulierten Monozyten produziert. Im Rahmen der erfindungsgemäßen Versuche wurde neben Calcidiol und Calcitriol als weitere Testsubstanz ein Vasokonstriktor eingesetzt.

Im Rahmen der Untersuchungen wurden zunächst humane Monozyten durch standardisierte und dem Fachmann grundsätzlich bekannte Methoden (Dichtegradientenzentrifugation oder magnetische Zellseparation unter Einsatz von *MicroBeads*^{®}) aus venösem Blut von Spendern isoliert. Anschließend wurden die gereinigten und hochvitalen Monozyten (95 %) in 48-Loch-Kulturplatten (10⁵/ml) in Gegenwart der Testsubstanzen inkubiert. Als Kontrollen bzw. Vergleichsversuche wurden Inkubationen der Monozyten in Gegenwart von Calcidiol bzw. Calcitriol sowie der weiteren Testsubstanzen jeweils alleine durchgeführt. Die Inkubation der Monozyten mit Calcidiol bzw. Calcitriol diente darüber hinaus dem generellen Nachweis von deren entzündungshemmender Wirkung. Darüber hinaus wurden Co-Inkubationen von Calcitriol bzw. Calcidiol in Kombination mit weiteren Testsubstanzen durchgeführt.

Während der Inkubation mit den Testsubstanzen erfolgte die Stimulation der Produktion von TNF-alpha (Tumornekrosefaktor-alpha) über einen Zeitraum von 20 Stunden mit Lipopolysacchariden (10 µg/ml LPS). Anschließend wurden die Überstände gewonnen und bis zur weiteren Analyse bei -80 °C gelagert. Zu Analysezwecken wurden die jeweiligen Kulturüberstände einem ELISA (*Enzyme-Linked Immunosorbent Assay*) unterzogen, dessen Durchführung dem Fachmann an sich bekannt ist, wobei die durch die Monozyten produzierte Menge an TNF-alpha gemessen wurde.

Bei den nachfolgend detailliert dargestellten Ergebnissen bzw. Untersuchungen sind die prozentualen Hemmwirkungen von Calcidiol bzw. Calcitriol, gegebenenfalls mit weiteren Testsubstanzen, auf die Produktion von TNF-alpha durch humane, mit LPS stimulierte Monozyten, im Vergleich zur Produktion von TNF-alpha durch Monozyten, welche durch LPS stimuliert, allerdings nicht in Gegenwart von Testsubstanzen inkubiert wurden, angegeben. Die Ergebnisse sind dargestellt als Hemmung in Prozent der LPS-induzierten Kontrolle ohne Wirkstoff aus gepoolten Messungen von mindestens zwei bis vier Experimenten (n = 8 bis 12). Für statistische Analysen wurde der nichtparametrische *Mann* & *Whitney-Test* herangezogen, insbesondere vor dem Hintergrund der Ermittlung einer Signifikanz im Vergleich zur LPS-Kontrolle, dem alleinigen Einsatz von Vitamin D bzw. von Calcidiol bzw. Calcitriol sowie dem alleinigen Einsatz der Testsubstanz. Ab einem p-Wert von < 0,05 gegenüber der jeweiligen Kontrolle bzw. dem jeweiligen Vergleichsansatz wird im Rahmen der vorliegenden Erfindung die Hemmwirkung als signifikant angesehen.

### a) Calcidiol bzw. Calcitriol als Entzündungshemmer

Zum Nachweis der entzündungshemmenden Wirkung von Calcidiol bzw. Calcitriol wurde der Einfluss auf die LPS-stimulierte Produktion von TNF-alpha durch humane Monozyten untersucht. Dazu wurde Calcidiol bzw. Calcitriol in jeweils separaten Experimentreihen in Konzentrationen von jeweils 0,1 ng/ml, 1 ng/ml, 10 ng/ml, 30 ng/ml, 50 ng/ml und 100 ng/ml eingesetzt. Die in Prozent angegebene Hemmung der TNF-alpha Produktion durch Calcitriol bzw. Calcidiol bezieht sich dabei auf die Menge an TNF-alpha, welche durch mit LPS stimulierte Monozyten ohne den Einsatz von Hemmstoffen produziert wurde.

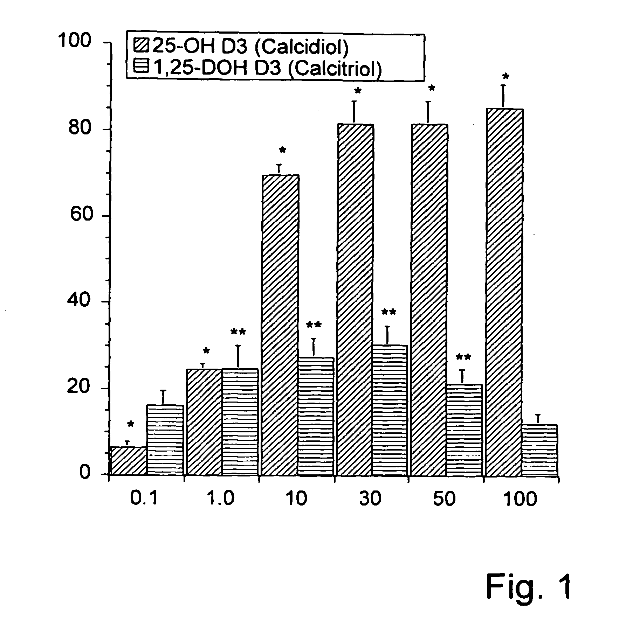

Die Ergebnisse zeigen überraschenderweise sowohl für Calcidiol als auch Calcitriol für Calcidiol eine signifikante starke Hemmung der Produktion von TNF-alpha, wobei die durch Calcidiol induzierte Hemmung diejenige von Calcitriol übersteigt. Die durch Calcidiol induzierte Hemmung von TNF-alpha variierte im Bereich von - 6,5 ± 1 % bei 0,1 ng/ml bis - 85,7 ± 5 % bei 100 ng/ml bei einem p-Wert von ≤ 0,0117. Somit kann die Hemmung der Produktion von TNF-alpha als signifikant angesehen werden. Mit Calcitriol konnte ebenfalls eine Hemmung von TNF-alpha erzielt werden, wobei diese mit Werten im Bereich von - 24,8 ± 5 % für 1 ng/ml und - 21,4 ± 3 % für 50 ng/ml mit einem Maximalwert der Hemmung von - 30,5 ± 4 % bei Einsatz von 30 ng/ml Calcitriol geringer ausfiel als die durch Calcidiol induzierte Hemmung. Die entsprechenden Ergebnisse sind Fig. 1 dargestellt, wobei die x-Achse die Konzentration an eingesetztem Calcidiol bzw. Calcitriol und die y-Achse den jeweiligen Mittelwert mit Angabe der Standardabweichung der prozentualen Hemmung im Vergleich zu der TNF-alpha-Kontrolle darstellt (mit *p = 0,0008 und **p < 0,017). Fig. 1 zeigt somit, dass im therapeutischen Konzentrationsbereich von Calcidiol (n = 8, 2 Experimente) und von Calcitriol (n = 8, 4 Experimente) die LPS-stimulierte Produktion von TNF-alpha in normalen Monozyten im Vergleich zur LPS-Kontrolle signifikant gehemmt wird.

Insgesamt konnte gezeigt werden, dass Vitamin D in seiner aktiven Form, insbesondere in Form von Calcidiol, aber auch in Form von Calcitriol, über eine eigenständige entzündungshemmende Wirkung verfügt. Im Gegensatz zu Calcidiol fällt die durch Calcitriol induzierte Hemmung von TNF-alpha bei normalen bzw. plasmarelevanten Konzentrationen, insbesondere im Bereich von 20 bis 55 ng/ml, wieder leicht ab. Steigende Calcitriol-Konzentrationen sind also insgesamt mit einem gewissen Rückgang der entzündungshemmenden Wirkung verbunden. Ohne sich hierbei auf diese Theorie beschränken zu wollen, spricht die abnehmende Entzündungshemmung bei höheren Konzentrationen von Calcitriol für eine Downregulation bzw. Herunterregulation des Vitamin D-Rezeptors durch höhere Konzentrationen an Calcitriol, so dass Calcitriol gewissermaßen imstande ist, seinen eigenen Rezeptor herunterzuregulieren.

Im Unterschied hierzu ist die Expression des Vitamin D-Rezeptors bei Vitamin D-Mangelsituationen induzierbar und wird durch eine Supplementierung oder Therapie mit Calcidiol nicht negativ beeinflusst.

Die obigen Ergebnisse erlauben die Schlussfolgerung, dass Vitamin D, insbesondere in seiner aktiven Form, für einen Einsatz im Rahmen der Behandlung von nicht-steroidpflichtigen, allergischen und entzündlichen Erkrankungen oder zur Steroidreduktion geeignet ist, wobei aufgrund der höheren antientzündlichen Wirkung Calcidiol bevorzugt wird. Calcidiol ist nämlich auch in höheren Konzentrationen in gesunden humanen Monozyten wirksam, was bislang im Stand der Technik bislang so nicht bekannt war.

### b) Vitamin D zur Intensivierung der Wirkung von Oxy- bzw. Xylometazolin bei oberen Atemwegserkrankungen

Hintergrund: Im Allgemeinen werden Atemwegserkrankungen in ätiopathologisch allergische bedingte (allergische Rhinitis), chronische, infektinduzierte (virale und bakterielle Rhinosinusitiden) und chronisch rezidivierende polypoide (chronisch rezidivierende polypoide Pansinusitis) obere Atemwegserkrankungen unterteilt.

Die Therapiekonzepte zu derartigen Atemwegserkrankungen umfassen im Allgemeinen zudem eine zeitlich begrenzte und rein symptomwirkrelevante topische Verabreichung sogenannter Antikongestiva, insbesondere von Xylo- oder Oxymetazolin, um eine Abschwellung der Nasenschleimhaut zu bewirken. Durch eine zu häufige und/oder zu lange andauernde Gabe derartiger Antikongestiva kann es zu einer schnellen Gewöhnung bzw. Resistenzbildung und im Ergebnis zu einer Wirkabschwächung kommen, was in einer sogenannten Previnitis oder *Rhinitis medicamentosa* resultieren kann. Darüber hinaus sehen die Therapiekonzepte häufig den Einsatz von Antiallergika, wie z. B. Dinatriumcromoglicinsäure, Nedocromil-Natrium und dergleichen, mit lediglich schwacher oder gänzlich fehlender entzündungshemmender Wirkung vor.

Die Leitlinienempfehlung sieht zudem für den saisonalen oder langfristigen Einsatz die Gabe topischer nasaler Kortikosteroide vor, welche aufgrund ihrer Nebenwirkungen häufig jedoch nur unregelmäßig eingesetzt werden. Darüber hinaus werden bei akuter viraler Rhinosinusitis alpha-1-Rezeptoragonisten zu Therapiezwecken eingesetzt, wobei diesen neben der antikongestiven Wirkung insbesondere in Kombination mit Ipratropiumbromid ein antirhinorrhoer Effekt zugeschrieben wird. Darüber hinaus haben im Zusammenhang mit persistierender allergischer Rhinitis klinische Studien ergeben, dass Xylometazolin im Vergleich zu Fluticasonfuroat ohne eine Zusatzwirkung bei Steroidvorbehandlung einen größeren antikongestiven Effekt besitzt. In-vitro-Studien haben zudem gezeigt, dass die Kombination von Dexpanthenol (5 Gew.-%, bezogen auf die Zusammensetzung) und Xylometazolin (0,05 Gew.-%, bezogen auf die Zusammensetzung) die Verträglichkeit von Xylometazolin in einem gewissen Umfang erhöht und toxische Effekte kontrolliert, jedoch ohne dass antientzündliche Zusatzeffekte bekannt sind.

Erfindungsgemäßer Ansatz: Im Rahmen der vorliegenden Erfindung wurde überraschend gefunden, dass Vitamin D, insbesondere Calcitriol bzw. dessen Präkursor Calcidiol, ein steroidartiges und stark entzündungshemmendes Profil zeigt. Insgesamt ergeben die erfindungsgemäßen Ergebnisse, dass dessen Einsatz bei mit nasaler Kongestion verbundenen Atemwegserkrankungen, insbesondere viralen, bakteriellen, vasomotorischen und allergischen Rhinitiden, wie beispielsweise Fließschnupfen, von besonderem Vorteil ist. Da die entzündungshemmenden Effekte von Calcidiol im Vergleich zu Calcitriol größer sind, wurden auch die Zusatzeffekte von Vitamin D in Form von Calcidiol und Xylometazolin im Rahmen der vorliegenden Erfindung untersucht.

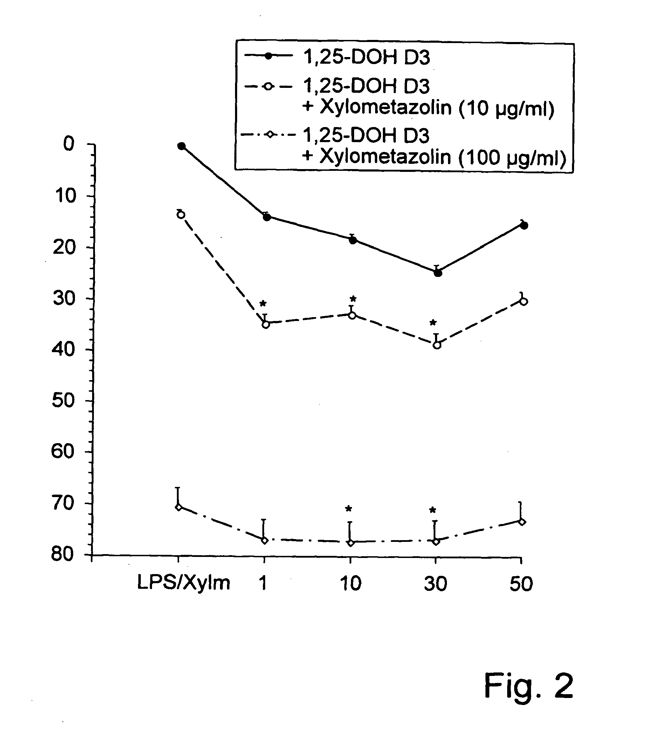

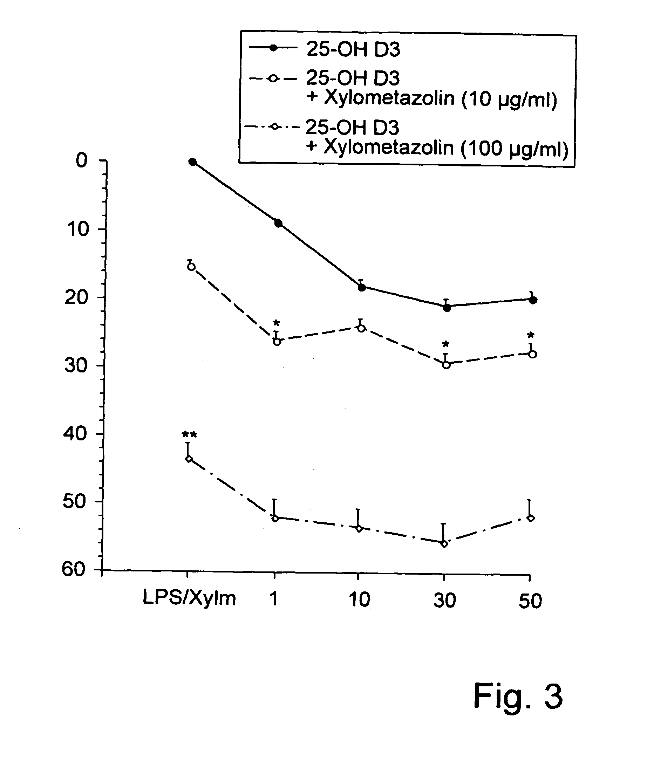

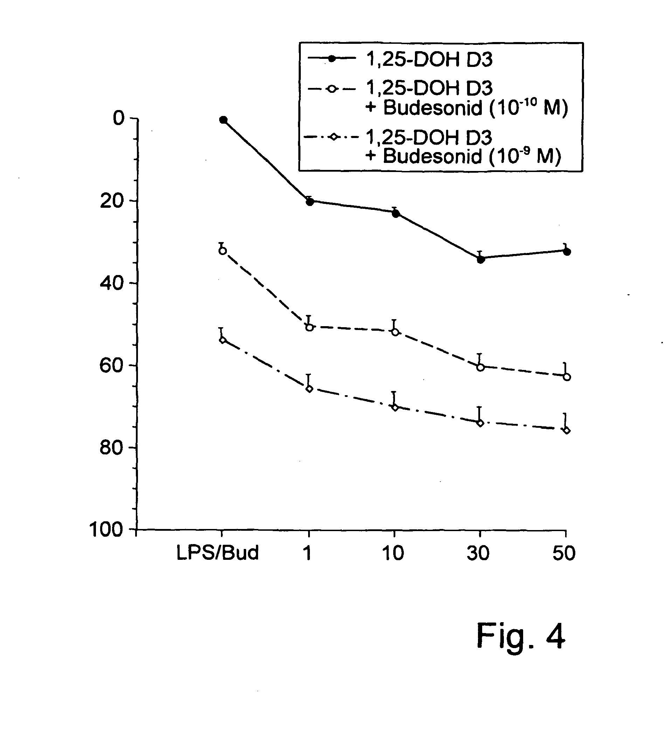

Im Rahmen dieser Untersuchungen wurde zunächst nachgewiesen, dass Xylometazolin eine gewisse eigenständige entzündungshemmende Wirkung vermittelt. Die im Rahmen der vorliegenden Erfindung durchgeführten Untersuchungen beschreiben in völlig überraschender Weise eine synergistische entzündungshemmende Wirkung sowohl für eine Kombination von Calcitriol und Xylometazolin (Fig. 2) als auch für eine Kombination von Calcidiol und Xylometazolin (Fig. 3).

Fig. 2 zeigt, dass die LPS-stimulierte Produktion von TNF-alpha durch steigende Konzentrationen von Calcitriol signifikant gehemmt wird (durchgezogene Linie). Co-Inkubation von Calcitriol mit einer subtherapeutischen Konzentration von Xylometazolin (10 µg/ml, gestrichelte Linie) führt zu einer signifikanten Zunahme der TNF-alpha-Hemmung im Vergleich zur Kontrolle (LPS/Xylometazolin 10 µg/ml alleine) und Calcitriol alleine. Die TNF-alpha-Hemmung nimmt weiter zu in Gegenwart einer hohen Konzentration von Xylometazolin (100 µg/ml, strichpunktierte Linie) und wird durch Co-Inkubation mit Calcitriol weiter verstärkt. In Fig. 2 zeigt die x-Achse die Konzentration an eingesetztem Calcitriol in ng/ml, und die y-Achse zeigt den jeweiligen Mittelwert mit Angabe der Standardabweichung der prozentualen Hemmung im Vergleich zu der TNF-alpha-Kontrolle (mit p < 0,021 vs. LPS-Kontrolle; *p ≤ 0.03 vs. Xylometazolin 10 µg/ml bzw. 100 µg/ml alleine).

Fig. 2 verdeutlicht somit, dass die LPS-stimulierte Produktion von TNF-alpha bei Co-Inkubation von Calcitriol mit einer subtherapeutischen Konzentration von Xylometazolin (10 mg/ml) zu einer signifikanten Zunahme der Hemmung der Produktion von TNF-alpha im Vergleich zur Kontrolle (LPS / Xylometazolin 10 mg/ml alleine) und zur alleinigen Inkubation mit Calcitriol führt. Darüber hinaus nimmt die TNF-alpha-Hemmung bei Co-Inkubation von Calcitriol sowie Xylometazolin in hoher Konzentration (100 µg/ml) weiter zu.

Fig. 3 zeigt, dass die LPS-stimulierte Produktion von TNF-alpha durch ansteigende Konzentrationen von Calcidiol signifikant gehemmt wird (durchgezogene Linie). Eine Co-Inkubation von Calcidiol mit einer Konzentration von Xylometazolin (10 µg/ml, gestrichelte Linie) führt zu einer signifikanten Zunahme der TNF-alpha-Hemmung im Vergleich zur Kontrolle (LPS / Xylometazolin 10 µg/ml) und Calcidiol alleine. Die TNF-alpha-Hemmung nimmt weiter zu in Gegenwart von höheren Konzentrationen an Xylometazolin (100 µg/ml, strichpunktierte Linie) und wird durch Co-Inkubation von Calcidiol weiter verstärkt. In Fig. 3 zeigt die x-Achse die Konzentration an eingesetztem Calcidiol in ng/ml, und die y-Achse zeigt den jeweiligen Mittelwert mit Angabe der Standardabweichung der prozentualen Hemmung im Vergleich zu der TNF-alpha-Kontrolle (mit p = 0,0008 vs. LPS-Kontrolle; *p < 0.021 vs. Xylometazolin 10 µg/ml alleine; **p = 0,0117 vs. Xylometazolin 10 µg/ml).

Aus Fig. 3 wird somit ersichtlich, dass die LPS-stimulierte TNF-alpha-Produktion in humanen Monozyten auch durch die Inkubation mit Calcidiol in ansteigenden Konzentrationen signifikant gehemmt wird. Die Co-Inkubation von Calcidiol mit einer subtherapeutischen Konzentration von Xylometazolin (10 µg/ml) führt zudem zu einer signifikanten Zunahme der TNF-alpha-Hemmung im Vergleich zur Kontrolle und Calcidiol alleine. Die TNF-alpha-Hemmung nimmt zudem bei einer Co-Inkubation von Calcidiol und einer hohen Konzentration von Xylometazolin (100 µg/ml) weiter zu.

Die erfindungsgemäß durchgeführten Untersuchungen zeigen somit in völlig überraschender Weise, dass sowohl Calcidiol als auch Calcitriol nach Co-Inkubation den entzündungshemmenden Effekt von Xylometazolin auf die untersuchte Hemmung von TNF-alpha um bis zu 300 %, bezogen auf die LPS-induzierte Stimulation der Produktion von TNF-alpha, in humanen Monozyten erhöhen können. Auf Basis der diesbezüglichen Ergebnisse ist herauszustellen, dass die untersuchten Kombinationen von Vitamin D mit Xylometazolin die entzündungshemmende und folglich auch die schleimhautabschwellende Wirkung von Xylometazolin in synergistischer Weise verstärken.

In Bezug auf die untersuchte Dosis/Wirkungs-Beziehung von Xylometazolin auf die Produktion von TNF-alpha führt der Zusatz von Calcidiol und Calcitriol zu einer Intensivierung der entzündungshemmenden Wirkung von Xylometazolin von etwa 500 %, bezogen auf den durch Xylometazolin allein hervorgerufenen Effekt, was bedeutet, dass eine etwa 5-fach verringerte Xylometazolin-Dosis in Gegenwart von Vitamin D einen vergleichbaren entzündungshemmenden Effekt hervorruft. Dieser erfindungsgemäße Aspekt erklärt, dass insbesondere bei Vitamin D-Mangelzuständen ein erhöhter Bedarf zur Entzündungshemmung besteht, welche bei zusätzlicher therapieinduzierter Abnahme der Wirkung nicht mit alpha-1-Sympathomimetika alleine gerade nicht wirksam behandelt werden kann.

Im Rahmen der vorliegenden Erfindung kann somit einem häufigen Problem des Standes des Technik erfolgreich entgegengetreten werden: Denn die oben bereits genannten Gewöhnungseffekte bzw. Resistenzbildungen und somit auch Wirkverluste machen generell beim Einsatz von Antikongestiva, insbesondere alpha-Symphathomimetika, niedrige Dosierungen erforderlich, welche nicht immer das erforderliche Wirkvermögen haben, die nasale Kongestion zu verbessern. Darüber hinaus wird mit den üblichen niedrigen Dosierungen nur eine unzureichende antientzündliche Wirkung erzielt. Insgesamt sind Zusammensetzungen des Standes der Technik daher üblicherweise nur zeitlich begrenzt zur primären symptomatischen, d. h. antikongestiven, nasalen Schleimhauttherapie geeignet. Aus diesem Grunde sind die verschiedenen, zugelassenen Präparate nicht als entzündungshemmende Therapeutika, insbesondere nicht zur Therapie der allergischen Rhinitis geeignet. Die im Rahmen der vorliegenden Erfindung überraschend gefunden Ergebnisse stellen eine Basis insbesondere für eine Langzeittherapie von Vitamin D in Kombination mit einem Wirkstoff aus der Klasse der alpha-1-Sympathomimetika, insbesondere Xylometazolin und/oder Oxymetazolin, zur Behandlung akuter und allergischer Erkrankungen der oberen Atemwege, insbesondere auch der allergischen Rhinitis, dar. Dabei kann auch der Einsatz von Zusatzstoffen, wie Dexpanthenol, in Betracht gezogen werden, um die Wirkung noch weiterführend zu verbessern. Insbesondere kann im Rahmen der vorliegenden Erfindung auch eine lokale Steroidersatztherapie bereitgestellt werden oder der Steroidbedarf in Sprayform oder durch ein nasales Beatmungsgerät (Pariboy) reduziert werden.

Zusätzlich ist die im Rahmen der vorliegenden Erfindung gefundene steroidartige Wirkung der vorgeschlagenen Kombinationen, insbesondere von Calcidiol und/oder Calcitriol, insbesondere Calcitriol, in Kombination mit einem alpha-1-Sympathomimetikum, auch dazu geeignet, die chronisch rezidivierende polypoide Pansinusitis zu behandeln.

### c) Vitamin D zur Intensivierung der Wirkung von Kortikosteroiden

Insbesondere wurde im Rahmen der vorliegenden Erfindung die direkte entzündungshemmende Wirkung von Vitamin D an isolierten Monozyten untersucht, welche den Vitamin D-Rezeptor exprimieren (Fig. 1).

Anschließend wurde die entzündungshemmende Wirkung einer Kombination auf Basis von Vitamin D, insbesondere Calcitriol, und Budesonid untersucht. Dazu wurde der hemmende Effekt von Calcitriol und Budesonid sowohl alleine als auch in Kombination auf die LPS-stimulierte Produktion von TNF-alpha in humanen Monozyten bestimmt. Die diesbezüglich erhaltenen Ergebnisse sind der nachfolgenden Tabelle 1 zu entnehmen.

**Tabelle 1: Effekt von Budesonid (Bud.) und Calcitriol (Vit. D) auf die LPS-stimulierte Produktion von TNF-alpha in humanen Monozyten**

| **Konz.** | **n** | **TNF-alpha (pg/10⁵ Monozyten)** | **Vergleich zur Kontrolle (%)** | **p-Wert** |
|---|---|---|---|---|
| ohne Stimulation | 8 | 756,1 ± 192 | - | - |
| 10 µg/ml LPS | 8 | 889,1 ± 52 | - | - |

| **Calcitriol alleine** | | | | |
|---|---|---|---|---|
| 1 ng/ml | 8 | 712 ± 31 | -20,0 | 0,0117 |
| 10 ng/ml | 8 | 687,5 ± 33 | -22,7 | 0,0008 |
| 30 ng/ml | 8 | 588,8 ± 14 | -33,8 | 0,0008 |
| 50 ng/ml | 8 | 605,6 ± 21 | -31,9 | 0,0008 |

| **Budesonid alleine** | | | | |
|---|---|---|---|---|
| 10⁻¹⁰ mol | 8 | 606,4 ± 110 | -31,8 | 0,0460 |
| 10⁻⁹ mol | 8 | 412,2 ± 22 | -53,6 | 0,0008 |

| **Budesonid in Kombination mit Calcitriol** | | | | |
|---|---|---|---|---|
| Bud. 10⁻¹⁰ mol und Vit. D 1 ng/ml | 8 | 439,4 ± 53 | -50,6 | 0,0008 |
| Bud. 10⁻¹⁰ mol und Vit. D 10 ng/ml | 8 | 430,0 ± 73 | -51,6 | 0,0008 |
| Bud. 10⁻¹⁰ mol und Vit. D 30 ng/ml | 8 | 354,4 ± 57 | -60,1 | 0,0008 |
| Bud. 10⁻¹⁰ mol und Vit. D 50 ng/ml | 8 | 333,4 ± 54 | -62,5 | 0,0008 |
| Bud.10⁻⁹ mol und Vit. D 1 ng/ml | 8 | 303,7 ± 12 | -65,5 | 0,0008 |
| Bud. 10⁻⁹ mol und Vit. D 10 ng/ml | 8 | 266,8 ± 22 | -70,0 | 0,0008 |
| Bud. 10⁻⁹ mol und Vit. D 30 ng/ml | 8 | 223.1 ± 18 | -73,8 | 0,0008 |
| Bud. 10⁻⁹ mol und Vit. D 50 ng/ml | 8 | 218,6 ± 27 | -75,5 | 0,0008 |

Die in Tabelle 1 aufgeführten Ergebnisse zeigen, dass sich überraschenderweise auf Basis einer Kombination von Budesonid und Calcitriol eine stärkere Hemmung der Produktion von TNF-alpha in humanen Monozyten erzielen lässt als mit Calcitriol bzw. Budesonid jeweils alleine, und zwar über die Wirkung der Einzelsubstanzen hinausgehend. Somit wurde im Rahmen der vorliegenden Erfindung auch eine synergistische Wirkung zwischen dem inhalativen Kortikosteroid Budesonid und Calcitriol gefunden, d. h. eine Zunahme der entzündungshemmenden Wirkung von Budesonid in Gegenwart von relevanten Konzentrationen an Calcitriol. Dies bedeutet, dass die Anwendung des erfindungsgemäßen Kombinationstherapeutikums als Co-Medikation zu einem Steroid gleichermaßen zu einer Verbesserung von dessen Wirksamkeit führt. Fig. 3 zeigt, dass die LPS-stimulierte TNF-alpha-Produktion durch ansteigende Konzentrationen von Calcitriol signifikant gehemmt wird (durchgezogene Linie). Co-Inkubation von Calcitriol mit einer niedrigeren Konzentration von Budesonid (10⁻¹⁰ mol/l, gestrichelte Linie) führt zu einer signifikanten Zunahme der TNF-alpha-Hemmung im Vergleich zur Kontrolle (LPS + Budesonid, Calcitriol oder Budesonid alleine). Diese Hemmeffekte nehmen weiter synergistisch zu nach Co-Inkubation von Calcitriol mit einer höheren Konzentration von Budesonid (10⁻⁹mol/l, strichpunktierte Linie). In Fig. 3 zeigt die x-Achse die Konzentration an eingesetztem Calcitriol in ng/ml, und die y-Achse zeigt den jeweiligen Mittelwert mit Angabe der Standardabweichung der prozentualen Hemmung im Vergleich zu der TNF-alpha-Kontrolle (mit p < 0,045 für Calcitriol, Budesonid, Budesonid + Calcitriol vs. LPS-Kontrolle; p < 0.016 für Budesonid 10⁻¹⁰ mol/l + Calcitriol vs. Calcitriol alleine; p = 0.0008 für Budesonid 10⁻⁹ mol/l + Calcitriol vs. Calcitriol alleine; p < 0.0275 für Budesonid 10⁻¹⁰ mol/l + Calcitriol vs. Budesonid 10⁻¹⁰ mol/l alleine; p < 0.0035 für Budesonid 10⁻⁹ mol/l + Calcitriol vs. Budesonid alleine).

### d) Anwendungs- und Wirksamkeitsstudien

Eine Patientengruppe aus 12 Probanden im Alter von 21 bis 68 Jahren mit akuten Rhinitiden wurde sowohl mit einer nichterfindungsgemäßen Zusammensetzung auf Basis einer wässrigen Lösung von Xylometazolin (Zusammensetzung 1) sowie mit erfindungsgemäßen Zusammensetzungen auf Basis von Xylometazolin einerseits und Vitamin D (Calcidiol) andererseits (Zusammensetzung 2) und einer erfindungsgemäßen Zusammensetzung auf Basis einer ternären Kombination von Xylometazolin, Vitamin D (Calcidiol) und Dexpanthenol (Zusammensetzung 3) behandelt. Die in Rede stehenden Zusammensetzungen wiesen Xylometazolin in Form von Xylometazolinhydrochlorid in einer Konzentration von 0,05 Gew.-%, bezogen auf die Zusammensetzung, auf. Calcidiol wurde in einer Menge von 0,001 Gew.-%, bezogen auf die Zusammensetzung, eingesetzt. Zudem wurde Dexpanthenol in der entsprechenden Zusammensetzung in einer Menge bzw. Konzentration von 2,5 Gew.-%, bezogen auf die Zusammensetzung, eingesetzt.

Sechs Patienten der in Rede stehenden Probandengruppe wurden jeweils mit dem Kombinationstherapeutikum 1 auf Basis von Xylometazolin und dem Kombinationstherapeutikum 2 auf Basis von Xylometazolin und Vitamin D behandelt. Dabei wurde jeweils eine Nasenseite mit der nichterfindungsgemäßen, Xylometazolinhydrochlorid allein enthaltenden Lösung gemäß Kombinationstherapeutikum 1 und die andere Nasenseite mit dem erfindungsgemäßen Kombinationstherapeutikum auf Basis von Kombinationstherapeutikum 2 (Xylometazolinhydrochlorid plus Vitamin D) behandelt. Weitere sechs Personen wurden in entsprechender Weise mit dem Kombinationstherapeutikum 1 sowie der Zusammensetzung 3 auf Basis von Xylometazolinhydrochlorid, Vitamin D und Dexpanthenol behandelt, wobei auch diesbezüglich jeweils eine Nasenseite mit dem nichterfindungsgemäßen Kombinationstherapeutikum (d. h. Kombinationstherapeutikum 1) und die andere Nasenseite mit dem erfindungsgemäßen Kombinationstherapeutikum (Kombinationstherapeutikum 3) behandelt wurde. Die Untersuchung der Probanden wurde mittels Rhinoskopie durchgeführt.

Bei sämtlichen Kombinationstherapeutika trat ein vasokonstriktorischer Effekt, bedingt durch Xylometazolin, auf. Auffallend und überraschend ist die Beobachtung, dass die von Xylometazolin ausgehende Reizwirkung bei Applikation der erfindungsgemäßen Kombinationstherapeutika gemäß Beispiel 2 und 3 nicht auftrat, woraus eine größere Compliance der Patienten resultierte. Dagegen wurden im Fall des nichterfindungsgemäßen Kombinationstherapeutikums gemäß Beispiel 1 Austrocknungen und entzündliche Irritationen der Nasenschleimhaut festgestellt. Bei den erfindungsgemäßen Kombinationstherapeutika konnte zudem beobachtet werden, dass der schleimhautabschwellende Effekt deutlicher hervortrat und zudem länger anhielt, so dass in Bezug auf die erfindungsgemäßen Kombinationstherapeutika eine deutlich verbesserte Wirkeffizienz vorliegt. In Bezug auf die erfindungsgemäßen Kombinationstherapeutika konnte zudem eine deutlich verringerte Entzündungsneigung der Nasenschleimhaut beobachtet werden, so dass die Erkrankung weniger stark ausgebildet wurde bzw. schneller abklingen bzw. abheilen konnte. Der diesbezügliche Effekt konnte bei dem erfindungsgemäßen Kombinationstherapeutikum auf Basis der ternären Kombination gemäß Beispiel 3 noch weiter verbessert werden.

Die Ergebnisse der Versuche zeigen einen deutlichen Synergismus der Wirkung des alpha-Sympathomimetikums Xylometazolin einerseits und dem Vitamin D andererseits, wobei dieser synergistische Effekt durch weitere Zugabe von Dexpanthenol noch weiter gesteigert werden kann; die erfindungsgemäßen pharmazeutischen Kombinationstherapeutika führen bei der Behandlung von akuten Rhinitiden zu einer eindrucksvollen Besserung, welche über das Ausmaß der Einzelwirkstoffe deutlich hinausgeht, was den synergistischen Effekt untermauert.

### Zusammenfassung:

Die im Rahmen der vorliegenden Erfindung durchgeführten Untersuchungen zeigen, dass die Stimulation des Vitamin D-Rezeptors mit den Agonisten Calcitriol und Calcidiol eine eigenständige, antientzündliche Wirkung auch bei relativ geringen Vitamin D-Konzentrationen von 30 ng/ml besitzt. Insbesondere vermittelt Calcitriol bzw. Calcidiol im Vergleich zur Kontrolle, d. h. von lediglich mit LPS-stimulierten humanen Monozyten ohne Co-Inkubation von Calcitriol bzw. Calcidiol, eine Hemmung der LPS-stimulierten Produktion von TNF-alpha von 30 bzw. 82 %.

Infolge des synergistischen Zusammenwirkens von Vitamin D bzw. dessen Metaboliten mit einem alpha-Sympathomimetikum wird im Rahmen der vorliegende Erfindung ein effizienter Therapieansatz insbesondere im Hinblick auf die Behandlung von Rhinitiden bereitgestellt, welcher sowohl zu einer Dosisreduktion des Sympathomimetikums als auch zu einem kürzeren Therapieverlauf führt. Zudem kann aufgrund der Interaktion von Vitamin D bzw. dessen Metaboliten mit insbesondere entzündungshemmenden Leitlinientherapeutika, wie Kortikosteroiden, welche beispielsweise im Rahmen der Behandlung von *Rhinitis allergica,* eingesetzt werden, ein deutlich verbesserter Therapieansatz bereitgestellt werden, auf dessen Basis eine Intensivierung bzw. Steigerung der Wirkeffizienz des im Rahmen der Leitlinientherapie verabreichten Therapeutikums ermöglicht wird, was gleichermaßen auf die völlig überraschend festgestellte, synergistisch zunehmende Entzündungshemmung durch den speziellen Einsatz von Vitamin D bzw. dessen Metaboliten zurückzuführen ist. Insgesamt ermöglichen die erfindungsgemäßen Therapiekonzepte eine effiziente Behandlung unterschiedlicher Formen von Erkrankungen der oberen Atemwege bzw. einer großen Vielzahl von Rhinitiden.

Die vorliegende Erfindung wird zudem durch die folgenden Aspekte näher beschrieben:

### Aspekt 1:

Vitamin D-Rezeptoragonisten (VDA), insbesondere 25-OH D₃ (Calcidiol) oder Analoga, zur Prophylaxe und Therapie der Entzündungshemmung bei allergischen, chronischen, infektiösen, exazerbierten Lokal- und Systemerkrankungen, mit dem Ziel der Reduktion des Therapiebedarfs bzw. Intensivierung der Hauptwirkung von anerkannten Leitlinientherapeutika, insbesondere unabhängig von einem Vit. D-Mangelzustand.

### Aspekt 2:

Vitamin D-Rezeptoragonisten (VDA), insbesondere 25-OH D₃ (Calcidiol) oder Analoga, bevorzugt zur prophylaktischen, lokalen, topischen und inhalativen Therapie von oberen und unteren Atemwegserkrankungen sowie zur lokalen Therapie chronisch entzündlicher Darmerkrankungen, insbesondere gelöst in dünndarmlöslichen Kapseln oder in Form von Pellets, insbesondere unabhängig von einem Vit. D-Mangelzustand.

### Aspekt 3:

Vitamin D-Rezeptoragonisten (VDA), insbesondere 25-OH D₃ (Calcidiol), 1,25-DOH D₃ (Calcitriol) oder deren Analoga, bevorzugt in lokaler, topischer Form bei Erkältungskrankheiten, chronisch obstruktiven Atemwegserkrankungen und/oder chronisch entzündlichen Darmerkrankungen zur Inaktivierung oder Hemmung der Vermehrung von viralen und bakteriellen Infekterregern, insbesondere mit dem Ziel, die Virulenz, Infektiosität, chronische persistierende Entzündungsreaktionen und somit den Therapiebedarf von Antibiotika, Kortikosteroiden und anderen Immunsupressiva, unabhängig von Vit. D-Mangelzuständen, zu reduzieren.

### Aspekt 4:

25-OH D₃ (Calcidiol) in Kombination mit 1,25-DOH D₃ (Calcitriol) zur Intensivierung der Wirkungen nach Aspekt 1 bis 3 durch eine Zunahme der Entzündungshemmung und einer Wirkverstärkung durch Reduktion der 1,25-DOH D₃ vermittelten Downregulation des Vitamin D₃-Rezeptors.

### Aspekt 5:

Vitamin D₃ wird eingesetzt als Tablette / dünndarmlösliche Kapsel (2 mal täglich 800 IE bis 2.000 IE), zur topisch-nasalen Therapie (50 bis 100 µg/Hub, 1-2 mal täglich) oder zur Inhalation in Suspension- oder Pulverform (250 µg/Hub bzw. 250 bis 500 µg/Pulver).

### Aspekt 6:

Verwendung von 25-OH D₃ und 1,25-DOH D₃ oder deren Analoga als Supplement in Verbindung mit 1,8-Cineol zur gegenseitigen Wirkverstärkung für die Prophylaxe und Behandlung saisonaler, d.h. typischer winterlicher Vitamin D₃-Mangelzustände, insbesondere zur Besserung der Infektabwehr und zur verbesserten entzündungshemmenden Therapie der Atemwege, mit dem Ziel, akute und chronische Exazerbationen bei chronisch obstruktiver Bronchitis, Asthma und chronisch entzündlichen und allergischen Erkrankungen, insbesondere auch bei chronischer Rhinosinusitis und allergischer Rhinitis, der oberen Atemwege zu reduzieren.

### Aspekt 7:

Verwendung von 25-OH D₃ und 1,25-DOH D₃ oder Analoga zur synergistischen Intensivierung der antiinfektiösen, antiallergischen, entzündungshemmenden und antioxidativen Wirkung von Monoterpenen, insbesondere von 1,8-Cineol (C) und L-Menthol in peroraler Darreichungs-form, vorzugsweise in Form von dünndarmlöslichen Kapseln (z. B. VD₃ 400 bis 600 IE + C 200 bis 300 mg).

### Aspekt 8:

Verwendung von 25-OH D₃ und 1,25-DOH D₃ oder deren Analoga zur lokalen-nasalen, inhalativen oder systemischen Therapie zur Prophylaxe und Therapie von viralen und bakteriellen Erkältungskrankheiten, insbesondere auch in Kombination mit 1,8-Cineol, Vitamin C, Vitamin E und Acetylsalicylsäure oder einem anderen nicht-steroidalen Antiphlogistikum (aus der Gruppe der NSAIDS) oder von Paracetamol zur Verstärkung von deren Wirksamkeit mit Besserung und Reduktion des Krankheitsverlaufs von Erkältungskrankheiten.

### Aspekt 9:

Verwendung von 25-OH D₃ und 1,25-DOH D₃ oder deren Analoga zur synergistischen Intensivierung der antiinfektiösen, antiallergischen, ent-zündungshemmenden und antioxidativen Wirkung von Mischterpenen, insbesondere von Pfefferminzölen (z. B. Colpermin^{®}) und standardisierten Mischterpenen (z. B. Gelomyrtol^{®}).

### Aspekt 10:

Verwendung von 25-OH D₃ oder Analoga, als Kombinationstherapeutikum, insbesondere in Form eines Kit als dünndarmlösliche Kapsel, mit einem Monoterpen, bevorzugt von 1,8-Cineol, einem Pfefferminzöl (z. B. Colpermin^{®}), oder einem standardisierten Mischterpen (z. B. Gelomyrtol^{®}) als Co-Medikation mit nasal-topischen Glukokortikosteroiden zur Intensivierung der Leitlinientherapie für obere Atemwegserkrankungen, insbesondere der akuten, allergischen, chronischen und chronisch rezidivierend polypoiden Rhinitis bzw. Rhinosinusitis.

### Aspekt 11:

Verwendung von 25-OH D₃ oder Analoga, als Kombinationstherapeutikum, insbesondere in Form eines Kit als Nasenspray, mit nasal-topischen Glukokortikosteroiden zur Intensivierung der Steroidwirkung bei oberen Atemwegserkrankungen, insbesondere der akuten, allergischen, chronischen und chronisch rezidivierenden, polypoiden Rhinitis bzw. Rhinosinusitis.

### Aspekt 12:

Verwendung von 1,25-DOH D₃, insbesondere von 25-OH D₃, oder deren Analoga als Kombinationstherapeutikum, insbesondere in Form eines Kit als Spray, bzw. Augentropfen, mit alpha-1-Sympathomimetika, insbesondere Xylometazolin, Xylometazolin+Dexpanthenol, Dexpanthenol, Oxymetazolin; Chromoglicerinsäure; Nedocromil; Vagolytika und/oder Antihistaminika für die lokale Therapie von Nase, Nasennebenhöhlen bzw. der Augen, und/oder für die Vermittlung bzw. Intensivierung abschwellender, antiviraler, antibakterieller und antientzündlicher, antipolypoider und antiallergischer Wirkungen.

### Aspekt 13:

Verwendung von 1,25-DOH D₃, insbesondere von 25-OH D₃, oder deren Analoga als Kombinationstherapeutikum, insbesondere in Form eines Kit als Spray in Form von alpha-1-Sympathomimetika, insbesondere Xylometazolin und Dexpanthenol oder Dexpanthenol alleine mit einer Erhöhung des Anteils von Dexpanthenol auf ≥ 20% mit dem Ziel einer ausreichenden entzündungshemmenden Wirkung für den Einsatz bei Rhinitis, allergischer Rhinitis und/oder chronischer Rhinosinusitis, gegebenenfalls mit *Polyposis nasi.*

### Aspekt 14:

Verwendung von 1,25-DOH D₃, insbesondere von 25-OH D₃, oder deren Analoga als Kombinationstherapeutikum, insbesondere in Form eines Kit als Spray für die lokale Therapie von Nase und Nasennebenhöhlen mit einem alpha-1-Sympathomimetikum, mit oder ohne Dexpanthenol, oder einem Glukokortikosteroid, zur Verhinderung oder Reduktion von Wirkverlusten durch Gewöhnungseffekte (Previnismus) mit anhaltender nasaler Kongestion mit Flußminderung.

### Aspekt 15:

Verwendung von 1,25-DOH D₃, insbesondere von 25-OH D₃, oder deren Analoga als Kombinationstherapeutikum, insbesondere in Form eines Kits als Spray mit einem alpha-1-Sympathomimetikum, insbesondere Xylometazolin, mit oder ohne Dexpanthenol oder Oxymetazolin, zur Behandlung von oberen Atemwegsobstruktionen sowie profibrotischen Entzündungsreaktionen bei schlafbezogenen Atmungsstörungen, insbesondere des obstruktiven und zentralen Schlafapnoesyndroms.

### Aspekt 16:

Verwendung von 1,25-DOH D₃, insbesondere von 25-OH D₃, oder deren Analoga als Kombinationstherapeutikum, insbesondere in Form eines Kits als dünndarmlösliche Kapsel, mit einem Monoterpen, bevorzugt von 1,8-Cineol, einem Pfefferminzöl (z. B. Colpermin^{®}) oder einem standardisierten Mischterpen (z. B. Gelomyrtol^{®}) als Co-Medikation der Leitlinientherapie (topisch-nasale Steroide, alpha-1-Sympathomimetika) für obere (akute, allergische und chronische Rhinitis bzw. Rhinosinusitis) und als Co-Medikation zur Intensivierung der Leitlinientherapie (LABA, ICS, LAMA, LABA+ICS, LABA+LAMA, LABA+ICS+LA-MA) für untere Atemwegserkrankungen (Asthma, COPD, chron. Lungenemphysem), insbesondere zur Induktion und/oder Verstärkung des steroidpermissiven Effekts, den Progress von Obstruktion und Emphysem, Exazerbationen und den Steroidbedarf zu reduzieren, sowie durch Zigarettenrauch oder Feinpartikel und andere Umweltnoxen assoziierte Atemwegserkrankungen.

### Aspekt 17:

Verwendung von 1,25-DOH D₃, insbesondere von 25-OH D₃, oder deren Analoga als Kombinationstherapeutikum, insbesondere in Form eines Kits zur Zusatztherapie, mit einem Monoterpen, bevorzugt 1,8-Cineol, systemischen Glukokortikosteroid (SCS), einer intravenösen Substitutionstherapie mit alpha-1-Antitrypsin (mit Vitamin D₃ 10.000 bis 15.000 IE / Woche) oder IgG-Immunglobulinen bei AK-Mangelsyndrom, sowie einem Phosphodiesteraseinhibitor (Roflumilast, Theophyllin) zur Behandlung von oberen bzw. unteren Atemwegserkrankungen (insbesondere chronischer Rhinosinusitis, allergischer Rhinitis, COPD und/oder Asthma), insbesondere mit der Indikation, die Therapieeffekte und Ziele der Leitlinientherapie zu intensivieren, insbesondere einer Induktion und/oder Verstärkung des steroidpermissiven Effekts, den Progress von Obstruktion und Emphysem, Exazerbationen und/oder den Steroidbedarf zu reduzieren und/oder zur Prophylaxe von mit Zigarettenrauch oder Feinpartikeln sowie mit anderen Umweltnoxen assoziierten Atemwegserkrankungen.

### Aspekt 18:

Verwendung von 1,25-DOH D₃, insbesondere von 25-OH D₃, oder Analoga als Kombinationstherapeutikum, insbesondere in Form eines Kits als Tablette oder Kapsel, in Verbindung mit Leukotrienrezeptorantagonisten, insbesondere Montelukast, Pranlukast, Zaforlukast, 5-Lipoxygenase-Hemmstoffen, oder Antiallergika bzw. Antihistaminika zur Intensivierung der Leitlinientherapieempfehlungen bei allergischen Erkrankungen, insbesondere des allergischen Asthmas, und/oder der allergischen Rhinitis und Konjunktivitis, Neurodermitis und von Nahrungsmittelallergien.

### Aspekt 19:

Verwendung von 1,25-DOH D₃, insbesondere von 25-OH D₃, oder deren Analoga als Kombinationstherapeutikum, insbesondere in Form eines Kits zur Inhalation, in Verbindung mit ICS, LABAs, LAMAs, LA-BA+ICS sowie LABA+ICS+LAMA, insbesondere mit dem Ziel der Verbesserung des Therapieeffekts der topisch eingesetzten Leitlinientherapie für untere Atemwegserkrankungen (siehe Aspekt 16).

### Aspekt 20:

Verwendung von 1,25-DOH D₃, insbesondere von 25-OH D₃, oder deren Analoga als Kombinationstherapeutikum, insbesondere in Form eines Kits als Zusatztherapie (+Vitamin D₃ 20 bis 30.000 IE / 2 Wochen), zusammen mit einer Anti-IgE-Therapie, insbesondere mit Omalizumab, zur Hemmung der Produktion von IgE-Antikörpern, vorzugsweise zum Schutz vor allergischen Sensibilisierungen bei gleichzeitiger Wirkverstärkung bei besserer Verträglichkeit mit eventueller Option zur Dosisreduktion kostenaufwendiger Therapien mit Anti-IgE.

### Aspekt 21:

Verwendung von Monoterpenen, insbesondere von 1,8-Cineol, mit oder ohne 25-OH D₃ oder 1,25-DOH D₃, zur Kompensation niedriger Wirkstoffspiegel von Vitamin D₃ oder auch Monoterpenen durch Co-Medikation beider Wirkstoffe als Kit zur Erhöhung der körpereigenen Vitamin D-Synthese oder durch Erhöhung der oralen Dosierung von dünndarmlöslichen Kapseln (3 x 400 mg) oder als intramuskuläre Depot-Injektion alle 2 bis 4 Wochen einer Kombination aus Vit.D3 (30.000/60.000 IE) und 1,8-Cineol (5/10 g) für die Prophylaxe, Therapie und Retardierung des Progress bei schweren chronisch-aktiven entzündlichen Darmerkrankungen sowie zur Reduktion von Exazerbationen, schwerer COPD, insbesondere des Lungenemphysems mit oder ohne respiratorischer Insuffizienz, schwerem Asthma, schweren Infektionserkrankungen, allergischen Reaktionen, zur Entzündungshemmung und Steroideinsparung, Osteoporose, Herzkreislauferkrankungen, Arteriosklerose, malignen Erkrankungen und Retardierung von Alterungsprozessen.

### Aspekt 22:

Verwendung nach einem der vorangehenden Aspekte zur Hemmung oder Modulation COPD-assoziierter und COPD-unabhängiger Alterungsprozesse, insbesondere mit dem Ziel, die Erkrankungshäufigkeit und -schwere zu reduzieren und die Lebenserwartung und -qualität zu bessern und den gesamten Alterungsprozess zu retardieren.

### Aspekt 23:

Verwendung von 1,25-DOH D₃, insbesondere von 25-OH D₃, oder deren Analoga als Kombinationstherapeutikum, insbesondere in Form eines Kits als dünndarmlösliche Kapsel mit einem Monoterpen, bevorzugt von 1,8-Cineol, oder einem ätherischen Mischöl zur Behandlung von oberen Atemwegsobstruktionen sowie profibrotischen Entzündungsreaktionen bei schlafbezogenen Atmungsstörungen, insbesondere des obstruktiven und zentralen Schlafapnoesyndroms.

### Aspekt 24:

Verwendung von 1,25-DOH D₃, insbesondere von 25-OH D₃, oder deren Analoga als Kombinationstherapeutikum, insbesondere in Form eines Kits als dünndarmlösliche Kapsel, in Verbindung mit einem Glukokortikosteroid, insbesondere Budesonid (Vitamin D₃+Budesonid), und einem Zytokininhibitor- oder Rezeptorantagonisten) oder anderen Biologika zur Intensivierung der Leitlinientherapie von chronisch entzündlichen Darmerkrankungen (IBD) mit Befall des Dünn- und Dickdarms, insbesondere von *Morbus Crohn* (CD) und *Colitis ulcerosa* (CU), hepatischen Autoimmunerkrankungen, wie bei primärbilärer Zirrhose, zur Prophylaxe und Erhöhung der Steroidwirkung bei IBD.

### Aspekt 25:

Verwendung von 1,25-DOH D₃, insbesondere von 25-OH D₃, oder Analoga als Kombinationstherapeutikum, insbesondere in Form eines Kits als dünndarmlösliche Kapsel, in Verbindung mit einem Monoterpen, bevorzugt von 1,8-Cineol (Vitamin D₃+1,8-Cineol), einem Pfefferminzöl, insbesondere Colpermin^{®} (Vitamin D₃+Colpermin^{®}), oder einem standardisiertem Mischöl (z. B. Vitamin D₃+Gelomyrtol^{®}) zur Intensivierung der Leitlinientherapie von chronisch entzündlichen Darmerkrankungen (IBD), einschließlich des Reizdarmsyndroms (IBS), *Morbus Crohn* (CD) und *Colitis ulcerosa* (CU) zur Prophylaxe, Remissionserhaltung bzw. Exazerbationsprophylaxe und Erhöhung der Entzündungshemmung bei Kindern unter 14 Jahren und Erwachsenen.

### Aspekt 26:

Prophylaxe chronisch entzündlicher Darmerkrankungen durch antioxidative Agentien, insbesondere eine Kombination von Vitamin D₃, Vitamin C, und/oder Vitamin E mit entweder Pfefferminzölen (z.B. Colpermin^{®}), Monoterpenen (1,8-Cineol, L-Menthol) oder Budesonid in dünndarmlöslichen Kapseln und in Form als Nahrungsmittelzusatz.

### Aspekt 27:

Verwendung von 1.25-DOH D₃, insbesondere von 25-OH D₃, oder deren Analoga als Kombinationstherapeutikum, insbesondere in Form eines Kits als dünndarmlösliche Kapsel, in Verbindung mit Budesonid oder anderen Glukokortikosteroiden, Monoterpenen, insbesondere von 1,8-Cineol, Misch- oder standardisierten Terpenen, insbesondere von Colpermin^{®} und Gelomyrtol^{®}, zur lokalen, systemischen und synergistischen Verstärkung der Leitlinientherapie der unter Aspekt 24 genannten Darmerkrankungen mit besonderem Fokus auf die klinische Überlegenheit durch Reduktion akuter, infektiöser, allergisch oder chronisch bedingter Rezidivhäufungen mit Besserung der Therapierefraktion durch einen neuen kausalen Ansatz zur kombinierten Behandlung der Lokal- und Systementzündung bei IBD.

## Patentansprüche

1. Kombinationstherapeutikum zur Verwendung bei der prophylaktischen und/oder therapeutischen topischen Behandlung von entzündlichen Erkrankungen der Nase und/oder der Nasennebenhöhlen, ausgewählt aus Rhinitis, Rhinosinusitis und Sinusitis,
wobei das Kombinationstherapeutikum intranasal applizierbar ist und/oder appliziert wird und
wobei das Kombinationstherapeutikum - in jeweils pharmazeutisch wirksamen Mengen - umfasst:
(a) mindestens einen Vitamin D-Rezeptoragonisten (VDA) in einer Menge im Bereich von 0,0005 Gew.-% bis 1,5 Gew.-%, bezogen auf das Kombinationstherapeutikum, und
(b) mindestens einen Vasokonstriktor in Form eines alpha-Sympathomimetikums oder dessen physiologisch unbedenkliche Salze in einer Menge im Bereich von 0,001 Gew.-% bis 2 Gew.-%, bezogen auf das Kombinationstherapeutikum.

2. Kombinationstherapeutikum zur Verwendung nach Anspruch 1,
wobei (a) der Vitamin D-Rezeptoragonist (VDA) ausgewählt ist aus der Gruppe von Verbindungen der Vitamin D-Gruppe, insbesondere Vitamin D₃ (Cholecalciferol) und/oder Vitamin D₂ (Ergocalciferol), vorzugsweise Vitamin D₃ (Cholecalciferol), und/oder wobei (a) der Vitamin D-Rezeptoragonist (VDA) 25-Hydroxy-Vitamin D₃ (Calcidiol) ist und/oder
wobei das Kombinationstherapeutikum (a) den Vitamin D-Rezeptoragonisten (VDA), insbesondere 25-Hydroxy-Vitamin D₃ (Calcidiol) und/oder 1,25-Dihydroxy-Vitamin D₃ (Calcitriol), in einer Menge im Bereich von 0,001 Gew.-% bis 1,25 Gew.-%, vorzugsweise im Bereich von 0,005 Gew.-% bis 1 Gew.-%, bevorzugt im Bereich von 0,01 Gew.-% bis 0,5 Gew.-%, besonders bevorzugt im Bereich von 0,02 Gew.-% bis 0,3 Gew.-%, ganz besonders bevorzugt im Bereich von 0,03 Gew.-% bis 0,15 Gew.-%, bezogen auf das Kombinationstherapeutikum, enthält.

3. Kombinationstherapeutikum zur Verwendung nach Anspruch 1 oder 2,
wobei (b) der Vasokonstriktor ein imidazolinbasiertes alpha-Sympathomimetikum ist und/oder
wobei das Kombinationstherapeutikum den (b) Vasokonstriktor in einer Menge im Bereich von 0,005 Gew.-% bis 1,5 Gew.-%, vorzugsweise im Bereich von 0,01 Gew.-% bis 1,2 Gew.-%, bevorzugt im Bereich von 0,02 Gew.-% bis 1,0 Gew.-%, besonders bevorzugt im Bereich von 0,03 Gew.-% bis 0,5 Gew.-%, ganz besonders bevorzugt im Bereich von 0,04 Gew.-% bis 0,2 Gew.-%, bezogen auf das Kombinationstherapeutikum, enthält.

4. Kombinationstherapeutikum zur Verwendung nach einem der vorangehenden Ansprüche,
wobei das Kombinationstherapeutikum (c) Pantothenol (Dexpanthenol) oder dessen physiologisch unbedenkliche Ester und/oder Pantothensäure oder deren physiologisch unbedenkliche Salze, vorzugsweise Pantothenol (Dexpanthenol) oder dessen physiologisch unbedenkliche Ester, bevorzugt Pantothenol (Dexpanthenol), enthält und/oder
wobei das Kombinationstherapeutikum (c) Pantothenol (Dexpanthenol) oder dessen physiologisch unbedenkliche Ester und/oder Pantothensäure oder deren physiologisch unbedenkliche Salze in einer Menge im Bereich von 0,01 Gew.-% bis 20 Gew.-%, insbesondere im Bereich von 0,1 Gew.-% bis 15 Gew.-%, vorzugsweise im Bereich von 0,5 Gew.-% bis 10 Gew.-%, bevorzugt im Bereich von 1 Gew.-% bis 7 Gew.-%, besonders bevorzugt im Bereich von 2 Gew.-% bis 6 Gew.-%, ganz besonders bevorzugt im Bereich von 3 Gew.-% bis 6 Gew.-%, bezogen auf das Kombinationstherapeutikum, enthält.

5. Kombinationstherapeutikum zur Verwendung nach einem der vorangehenden Ansprüche,
wobei das Kombinationstherapeutikum (f) mindestens einen weiteren pharmakologisch wirksamen Inhaltsstoff und/oder Wirkstoff enthält,
insbesondere wobei (f) der weitere pharmakologisch wirksame Inhaltsstoff und/oder Wirkstoff ein Antiallergikum, insbesondere Antihistaminikum und/oder Mastzellstabilisator, ist, insbesondere wobei das Antiallergikum ausgewählt ist aus der Gruppe von Azelastin, Levocabstin, Cromoglicinsäure und Nedocromil sowie Mischungen und Kombinationen der vorgenannten Verbindungen, oder
insbesondere wobei (f) der weitere pharmakologisch wirksame Inhaltsstoff und/oder Wirkstoff ein insbesondere topisch applizierbares, vorzugsweise nasal applizierbares Kortikosteroid, insbesondere Glukokortikosteroid, ist, insbesondere wobei das Glukokortikosteroid ausgewählt ist aus der Gruppe von Dexamethason, Budesonid, Prednison, Prednisolon, Betamethason, Rimexolon und Fluorometholon sowie Mischungen und Kombinationen der vorgenannten Verbindungen.

6. Kombinationstherapeutikum zur Verwendung nach Anspruch 5, wobei das Kombinationstherapeutikum den (f) weiteren pharmakologisch wirksamen Inhaltsstoff und/oder Wirkstoff in einer Menge im Bereich von 0,001 Gew.-% bis 5 Gew.-%, insbesondere im Bereich von 0,01 Gew.-% bis 2 Gew.-%, vorzugsweise im Bereich von 0,1 Gew.-% bis 1 Gew.-%, bezogen auf das Kombinationstherapeutikum, enthält.

7. Kombinationstherapeutikum zur Verwendung nach einem der vorangehenden Ansprüche,
wobei das Kombinationstherapeutikum als insbesondere wässrige Zusammensetzung vorliegt und/oder wobei das Kombinationstherapeutikum wässrig basiert ist und/oder wässrig formuliert vorliegt, insbesondere in Form einer wässrigen Lösung oder wässrigen Solubilisierung und/oder Emulsion, oder aber wobei das Kombinationstherapeutikum als Trockenformulierung, insbesondere als Pulver, vorzugsweise Sprühpulver, vorliegt und/oder
wobei das Kombinationstherapeutikum insbesondere im Fall der wässrigen Formulierung einen pH-Wert im Bereich von 5,0 bis 7,0, insbesondere im Bereich von 5,1 bis 6,5, vorzugsweise im Bereich von 5,2 bis 6,0, aufweist.

8. Kombinationstherapeutikum zur Verwendung nach einem der vorangehenden Ansprüche, in Form eines Kits.

9. Kombinationstherapeutikum zur Verwendung nach einem der Ansprüche 1 bis 8
zur prophylaktischen und/oder therapeutischen topischen Behandlung von *Rhinitis acuta,* und/oder zur prophylaktischen und/oder therapeutischen topischen Behandlung von *Rhinitis acuta, Rhinitis allergica, Rhinitis atrophicans, Rhinitis hyperplastica* oder *hypertrophicans, Rhinitis mutilans, Rhinitis nervosa* oder *vasomotorica* oder *Rhinitis pseudomembranacea,* vorzugsweise *Rhinitis acuta* oder *Rhinitis allergica* oder
zur prophylaktischen und/oder therapeutischen topischen Behandlung von polypoider Rhinitis, insbesondere chronischer und/oder chronisch rezidivierender Rhinitis, oder
zur Intensivierung der Wirksamkeit und/oder Wirksamkeitssteigerung und/oder Dosisreduktion von Steroiden bei der akuten, allergischen, chronischen und/oder chronisch rezidivierenden, polypoiden Rhinitis und/oder Rhinosinusitis, insbesondere wobei das Steroid ein insbesondere topisch applizierbares, vorzugsweise nasal applizierbares Kortikosteroid, insbesondere Glukokortikosteroid, ist, vorzugsweise ausgewählt aus der Gruppe von Dexamethason, Budesonid, Prednison, Prednisolon, Betamethason, Rimexolon und Fluorometholon sowie Mischungen und Kombinationen der vorgenannten Verbindungen, und/oder insbesondere wobei das Kombinationstherapeutikum als flüssige Formulierung, insbesondere Sprühformulierung, vorzugsweise Nasenspray, vorliegt.

10. Kombinationstherapeutikum zur Verwendung nach einem der Ansprüche 1 bis 8 zur Wirkungsvermittlung und/oder Intensivierung der Wirksamkeit und/oder Wirksamkeitssteigerung abschwellender, antiviraler, antibakterieller, antientzündlicher, antipolypoider und/oder antiallergischer Wirkstoffe, insbesondere wobei das Kombinationstherapeutikum als flüssige Formulierung, insbesondere Sprüh- oder Tropfformulierung, vorzugsweise Nasenspray, vorliegt.

11. Kombinationstherapeutikum zur Verwendung nach einem der Ansprüche 1 bis 8 zur Bereitstellung einer entzündungshemmenden Wirkung für den Einsatz bei Rhinitis, allergischer Rhinitis und chronischer Rhinosinusitis, gegebenenfalls in Kombination mit *Polyposis nasi,* insbesondere wobei das Kombinationstherapeutikum zudem Dexpanthenol enthält und/oder insbesondere wobei das Kombinationstherapeutikum als flüssige Formulierung, insbesondere Sprühformulierung, vorzugsweise Nasenspray, vorliegt.

12. Kombinationstherapeutikum nach zur Verwendung einem der Ansprüche 1 bis 8 zur Verhinderung oder Reduktion von Wirkverlusten durch Gewöhnungseffekte und/oder Previnismus), insbesondere mit anhaltender nasaler Kongestion und/oder mit Flussminderung, insbesondere wobei das Kombinationstherapeutikum mindestens ein Steroid, vorzugsweise topisch applizierbares, vorzugsweise nasal applizierbares Kortikosteroid, insbesondere Glukokortikosteroid enthält und/oder wobei das Kombinationstherapeutikum als flüssige Formulierung, insbesondere Sprühformulierung, vorzugsweise Nasenspray, vorliegt.

13. Applikationsvorrichtung für die intranasale Applikation, bevorzugt in Form eines Behältnisses mit Tropf- oder Sprüheinrichtung, enthaltend ein Kombinationstherapeutikum nach einem der vorangehenden Ansprüche.

14. Applikationsvorrichtung nach Anspruch 13,
wobei die Applikationsvorrichtung eine Sprüheinrichtung zur gleichförmigen Ausbringung der Zusammensetzung in einer Menge pro Sprühstoß im Bereich von 20 µl bis 300 µl, insbesondere im Bereich von 30 µl bis 200 µl, vorzugsweise im Bereich von 40 al bis 100 µl, aufweist oder
wobei die Applikationsvorrichtung eine Tropfeinrichtung zur gleichförmigen Ausbringung der Zusammensetzung in einer Menge pro Tropfen im Bereich von 10 µl bis 200 µl, insbesondere im Bereich von 20 µl bis 150 µl, vorzugsweise im Bereich von 15 µl bis 100 µl, aufweist.

## Claims

1. Combination therapeutic agent for use in the prophylactic and/or therapeutic topical treatment of inflammatory diseases of the nose and/or sinuses, selected from rhinitis, rhinosinusitis and sinusitis,
whereby the combination therapeutic agent is intranasally applicable and/or is applied, and
whereby the combination therapeutic agent - in respectively pharmaceutically effective amounts - comprises:
(a) at least one vitamin D receptor agonist (VDA) in an amount ranging from 0.0005 wt.-% to 1.5 wt.-%, based on the combination therapeutic agent, and
(b) at least one vasoconstrictor in the form of an alpha sympathomimetic agent or its physiologically acceptable salts in an amount ranging from 0.001 wt .-% to 2 wt .-%, based on the combination therapeutic agent.

2. Combination therapeutic agent for use according to claim 1,
whereby (a) the vitamin D receptor agonist (VDA) is selected from the group of compounds of the vitamin D group, in particular vitamin D₃ (cholecalciferol) and/or vitamin D₂ (ergocalciferol), preferably vitamin D₃ (cholecalciferol), and/or whereby (a) the vitamin D receptor agonist (VDA) is 25-hydroxy-vitamin D₃ (calcidiol), and/or
whereby the combination therapeutic agent contains (a) the vitamin D receptor agonist (VDA), in particular 25-hydroxy-vitamin D₃ (calcidiol) and/or 1,25-dihydroxy-vitamin D₃ (calcitriol), in an amount in the range of 0.001 wt .-% to 1.25 wt .-%, preferably in the range of 0.005 wt .-% to 1 wt .-%, more preferably in the range of 0.01 wt.-% to 0.5 wt.-%, even more preferably in the range of 0.02 wt.-% to 0.3 wt.-%, most preferably in the range of 0.03 wt .-% to 0.15 wt .-%, based on the combination therapeutic agent.

3. Combination therapeutic agent for use according to claim 1 or 2,
whereby (b) the vasoconstrictor is an imidazoline-based alpha sympathomimetic substance and/or
whereby the combination therapeutic agent contains (b) a vasoconstrictor in an amount in the range of 0.005 wt.-% to 1.5 wt.-%, preferably in the range of 0.01 wt.-% to 1.2 wt .-%, more preferably in the range of 0.02 wt .-% to 1.0 wt .-%, even more preferably in the range of 0.03 wt.-% to 0.5 wt.-%, most preferably in the range of 0.04 wt.-% to 0.2 wt.-%, based on the combination therapeutic agent.

4. Combination therapeutic agent for use according to any one of the preceding claims,
whereby the combination therapeutic agent contains (c) pantothenol (dexpanthenol) or its physiologically acceptable esters and/or pantothenic acid or its physiologically acceptable salts, preferably pantothenol (dexpanthenol) or its physiologically acceptable esters, more preferably pantothenol (dexpanthenol), and/or
whereby the combination therapeutic agent contains (c) pantothenol (dexpanthenol) or its physiologically acceptable esters and/or pantothenic acid or its physiologically acceptable salts in an amount in the range of 0.01 wt .-% to 20 wt .-%, particularly in the range of 0.1 wt.-% to 15 wt .-%, preferably in the range of 0.5 wt .-% to 10 wt.-%, more preferably in the range of 1 wt .-% to 7 wt.-%, even more preferably in the range of 2 wt.-% to 6 wt.-%, most preferably in the range of 3 wt.-% to 6 wt .-%, based on the combination therapeutic agent.

5. Combination therapeutic agent for use according to any one of the preceding claims,
whereby the combination therapeutic agent contains (f) at least one further pharmacologically active ingredient and/or active agent,
in particular whereby (f) the further pharmacologically active ingredient and/or active agent is an anti-allergic agent, in particular antihistamine and/or mast cell stabiliser, in particular whereby the anti-allergic agent is selected from the group of azelastine, levocabastine, cromolyn and nedocromil, as well as mixtures and combinations of the aforementioned compounds, or
in particular whereby (f) the further pharmacologically active ingredient and/or active agent is a particularly topical administrable agent, preferably nasally-administrable corticosteroid, particularly glucocorticosteroid, in particular whereby the glucocorticosteroid is selected from the group of dexamethasone, budesonide, prednisone, prednisolone, betamethasone, rimexolone and fluorometholone, as well as mixtures and combinations of the aforementioned compounds.

6. Combination therapeutic agent for use according to claim 5,
whereby the combination therapeutic agent contains (f) further pharmacologically active ingredient and/or active agent in an amount in the range of 0.001 wt.-% to 5 wt .-%, in particular in the range of 0.01 .-% to 2 wt.-%, preferably in the range of 0.1 wt.-% to 1 wt.-%, based on the combination therapeutic agent.

7. Combination therapeutic agent for use according to one of the preceding claims
whereby the combination therapeutic agent is present particularly as an aqueous composition and/or whereby the combination therapeutic agent is aqueous-based and/or aqueous-formulated, particularly in the form of an aqueous solution or aqueous solubilisation and/or emulsion, or whereby the combination therapeutic agent is present as a dry formulation, in particular as a powder, preferably spray-dried powder, and/or
whereby the combination therapeutic agent, particularly in the case of the aqueous formulation, has a pH value in the range of 5.0 to 7.0, in particular in the range of 5.1 to 6.5, preferably in the range of 5.2 to 6.0.

8. Combination therapeutic agent for use according to one of the preceding claims in the form of a kit.

9. Combination therapeutic agent for use according to one of the claims 1 to 8
for the prophylactic and/or therapeutic topical treatment of *rhinitis acuta* and/or for the prophylactic and or therapeutic topical treatment of *rhinitis acuta, rhinitis allergica, rhinitis atrophicans, rhinitis hyperplastica or hypertrophica, rhinitis mutilans, rhinitis nervosa* or *vasomotoria* or *rhinitis pseudomembranosa.* preferably *rhinitis acuta* or *rhinitis allergica,* or
for the prophylactic and/or therapeutic topical treatment of polypoid rhinitis, in particular chronic and/or chronic recurrent rhinitis, or
to intensify the effectiveness and/or increase the effectiveness and/or reduce the dose of steroids in the case of acute, allergic, chronic and/or chronic recurrent polypoid rhinitis and/or rhinosinusitis, in particular whereby the steroid is an especially topically administrable, preferably nasally administrable corticosteroid, in particular glucocorticosteroid, preferably selected from the group of dexamethasone, budesonide, prednisone, prednisolone, betamethasone, rimexolone and fluorometholone, as well as mixtures and combinations of the aforementioned compounds, and/or in particular whereby the therapeutic combination is present as an aqueous formulation, in particular a spray formulation, preferably a nasal spray.

10. Combination therapeutic agent for use according to any one of the claims 1 to 8 for effective mediation and/or to intensify the effectiveness and/or increase the effectiveness of decongestant, antiviral, antibacterial, anti-inflammatory, anti-polypoidal and/or anti-allergic agents, in particular whereby the combination therapeutic agent is present as an aqueous formulation, in particular a spray or drip formulation, preferably a nasal spray.

11. Combination therapeutic agent for use according to any one of the claims 1 to 8 to provide an anti-inflammatory effect for use in rhinitis, allergic rhinitis and chronic rhinosinusitis, optionally in combination with *polyposis nasi,* in particular whereby the combination therapeutic agent also contains dexpanthenol and/or in particular whereby the combination therapeutic agent is present as an aqueous formulation, in particular a spray or drip formulation, preferably a nasal spray.

12. Combination therapeutic agent for use according to any one of the claims 1 to 8 for the prevention or reduction of losses in effectiveness through habituation and/or rhinitis medicamentosa, especially with persistent nasal congestion and/or flow reduction, in particular where the combination therapeutic agent contains at least one steroid, preferably topically administrable, preferably nasally administrable, corticosteroid, particularly glucocorticosteroid and/or whereby the combination therapeutic agent is present as an aqueous formulation, in particular a spray or drip formulation, preferably a nasal spray

13. Application device for intranasal administration, preferably in the form of a container with a drip or spray device containing a combination therapeutic agent according to any one of the preceding claims.

14. Application device according to claim 13,
whereby the application device is a spray device for the uniform application of the compound in an amount per push in the range of 20 µl to 300 µl, in particular in the range of 30 µl to 200 µl, preferably in the range of 40 µl to 100 µl, or
whereby the application device comprises a drip device for the uniform application of the compound in an amount per drop in the range of 10 µl to 200 µl, in particular in the range of 20 µl to 150 µl, preferably in the range of 15 µl to 100 µl.

## Revendications

1. Agent thérapeutique en association pour une utilisation lors du traitement topique prophylactique et/ou thérapeutique de maladies inflammatoires du nez et/ou des sinus paranasaux, choisies parmi la rhinite, la rhinosinusite et la sinusite,
l'agent thérapeutique en association pouvant être administré et/ou étant administré par voie intranasale,
l'agent thérapeutique en association comprenant, dans chaque cas en des quantités à effet pharmaceutique :
(a) au moins un agoniste des récepteurs de la vitamine D (VDA) en une quantité comprise dans la plage de 0,0005 % en poids à 1,5 % en poids, par rapport à l'agent thérapeutique en association, et
(b) au moins un vasoconstricteur sous forme d'un alpha-sympathicomimétique ou de ses sels physiologiquement inoffensifs, en une quantité comprise dans la plage de 0,001 % en poids à 2 % en poids, par rapport à l'agent thérapeutique en association.

2. Agent thérapeutique en association pour l'utilisation selon la revendication 1,
dans lequel (a) l'agoniste des récepteurs de la vitamine D (VDA) est choisi dans le groupe des composés du groupe de la vitamine D, en particulier la vitamine D₃ (cholécalciférol) et/ou la vitamine D₂ (ergocalciférol), de préférence la vitamine D₃ (cholécalciférol), et/ou (a) l'agoniste des récepteurs de la vitamine D (VDA) est la 25-hydroxy-vitamine D₃ (calcidiol), et/ou
l'agent thérapeutique en association contenant (a) les agonistes des récepteurs de la vitamine D (VDA), en particulier la 25-hydroxy-vitamine D₃ (calcidiol) et/ou la 1,25-dihydroxy-vitamine D₃ (calcitriol), en une quantité comprise dans la plage de 0,001 % en poids à 1,25 % en poids, de préférence dans la plage de 0,005 % en poids à 1 % en poids, d'une manière préférée dans la plage de 0,01 % en poids à 0,5 % en poids, d'une manière particulièrement préférée dans la plage de 0,02 % en poids à 0,3 % en poids, d'une manière tout particulièrement préférée dans la plage de 0,03 % en poids à 0,15 % en poids, par rapport à l'agent thérapeutique en association.

3. Agent thérapeutique en association pour l'utilisation selon la revendication 1 ou 2,
dans lequel (b) le vasoconstricteur est un alpha-sympathicomimétique à base d'imidazoline et/ou
l'agent thérapeutique en association contenant (b) le vasoconstricteur en une quantité comprise dans la plage de 0,005 % en poids à 1,5 % en poids, de préférence dans la plage de 0,01 % en poids à 1,2 % en poids, d'une manière préférée dans la plage de 0,02 % en poids à 1,0 % en poids, d'une manière particulièrement préférée dans la plage de 0,03 % en poids à 0,5 % en poids, d'une manière tout particulièrement préférée dans la plage de 0,04 % en poids à 0,2 % en poids, par rapport à l'agent thérapeutique en association.

4. Agent thérapeutique en association pour l'utilisation selon l'une des revendications précédentes,
l'agent thérapeutique en association contenant (c) du pantothénol (dexpanthénol) ou ses esters physiologiquement inoffensifs et/ou de l'acide pantothénique ou ses sels physiologiquement inoffensifs, de préférence le pantothénol (dexpanthénol) ou ses sels physiologiquement inoffensifs, de préférence le pantothénol (dexpanthénol), et/ou
l'agent thérapeutique en association contenant (c) le pantothénol (dexpanthénol) ou ses esters physiologiquement inoffensifs et/ou l'acide pantothénique ou ses sels physiologiquement inoffensifs en une quantité comprise dans la plage de 0,01 % en poids à 20 % en poids, en particulier dans la plage de 0,1 % en poids à 15 % en poids, de préférence dans la plage de 0,5 % en poids à 10 % en poids, d'une manière préférée dans la plage de 1 % en poids à 7 % en poids, d'une manière particulièrement préférée dans la plage de 2 % en poids à 6 % en poids, d'une manière tout particulièrement préférée dans la plage de 3 % en poids à 6 % en poids, par rapport à l'agent thérapeutique en association.

5. Agent thérapeutique en association pour l'utilisation selon l'une des revendications précédentes,
l'agent thérapeutique en association (f) contenant au moins un autre constituant et/ou principe actif à effet pharmacologique,
en particulier dans lequel (f) l'autre constituant et/ou principe actif à effet pharmacologique est un antiallergique, en particulier un antihistaminique et/ou un stabilisant des mastocytes, en particulier dans lequel l'antiallergique est choisi dans le groupe de l'azélastine, de la lévocabstine, de l'acide cromoglicique et du néodocromil, ainsi que des mélanges et combinaisons des composés mentionnés ci-dessus, ou
en particulier dans lequel (f) l'autre constituant et/ou principe actif à effet pharmacologique est un corticostéroïde pouvant être administré par voie topique, en particulier pouvant être administré par voie nasale, en particulier un glucocorticostéroïde, en particulier dans lequel le glucocorticostéroïde est choisi dans le groupe de la dexaméthasone, du budésonide, la prednisone, la prednisolone, la bêtaméthasone, la riméxolone et la fluorométholone, ainsi que les mélanges et combinaisons des composés mentionnés ci-dessus.

6. Agent thérapeutique en association pour l'utilisation selon la revendication 5, l'agent thérapeutique en association contenant (f) l'autre constituant et/ou principe actif à effet pharmacologique en une quantité comprise dans la plage de 0,001 % en poids à 5 % en poids, en particulier dans la plage de 0,01 % en poids à 2 % en poids, de préférence dans la plage de 0,1 % en poids à 1 % en poids, par rapport à l'agent thérapeutique en association.

7. Agent thérapeutique en association pour l'utilisation selon l'une des revendications précédentes,
l'agent thérapeutique en association se présentant en particulier sous forme d'une composition aqueuse, et/ou l'agent thérapeutique en association étant à base aqueuse et/ou se présentant sous forme d'une formulation aqueuse, en particulier sous forme d'une solution aqueuse ou d'une solubilisation et/ou d'une émulsion aqueuse, ou encore l'agent thérapeutique en association se présentant cependant sous forme d'une formulation sèche, en particulier d'une poudre, de préférence d'une poudre aérosol, et/ou
l'agent thérapeutique en association présentant, en particulier dans le cas de la formulation aqueuse, un pH compris dans la plage de 5,0 à 7,0, en particulier dans la plage de 5,1 à 6,5, de préférence dans la plage de 5,2 à 6,0.

8. Agent thérapeutique en association pour l'utilisation selon l'une des revendications précédentes, sous forme d'une trousse.

9. Agent thérapeutique en association pour l'utilisation selon l'une des revendications 1 à 8,
pour le traitement topique, prophylactique et/ou thérapeutique, de *Rhinitis acuta,* et/ou pour le traitement topique prophylactique et/ou thérapeutique de *Rhinitis acuta,* de *Rhinitis allergica,* de *Rhinitis atrophicans,* de *Rhinitis hyperplastica* ou *hypertrophicans,* de *Rhinitis mutilans,* de *Rhinitis nervosa* ou *vasomotorica* ou de *Rhinitis pseudomembranacea,* de préférence de *Rhinitis acuta* ou de *Rhinitis allergica,* ou
pour le traitement topique prophylactique et/ou thérapeutique de la rhinite polypoïde, en particulier de la rhinite chronique et/ou récidivante chronique, ou
pour l'intensification de l'efficacité et/ou l'augmentation de l'efficacité et/ou la réduction de la dose de stéroïdes en présence d'une rhinite polypoïde et/ou d'une rhinosinusite aiguë, allergique, chronique et/ou récidivante chronique, le stéroïde étant en particulier un corticostéroïde pouvant être administré par voie topique, de préférence pouvant être administré par voie nasale, en particulier un glucocorticostéroïde, choisi de préférence dans le groupe de la dexaméthasone, du budésonide, de la prednisone, de la prednisolone, de la bêtaméthasone, de la riméxolone et de la fluorométholone, ainsi que des mélanges et combinaisons des composés mentionnés ci-dessus, et/ou en particulier l'agent thérapeutique en association se présentant sous forme d'une formulation liquide, en particulier d'une formulation en aérosol, de préférence d'un spray nasal.

10. Agent thérapeutique en association pour l'utilisation selon l'une des revendications 1 à 8 pour assurer un effet et/ou intensifier l'effet et/ou augmenter l'effet de principes actifs décongestionnants, antiviraux, antibactériens, anti-inflammatoires, antipolypoïdes et/ou antiallergiques, l'agent thérapeutique en association se présentant en particulier sous forme d'une formulation liquide, en particulier d'une formulation en aérosol ou en gouttes, de préférence sous forme d'un spray nasal.

11. Agent thérapeutique en association pour l'utilisation selon l'une des revendications 1 à 8 pour assurer un effet anti-inflammatoire dans le cadre d'une utilisation lors d'une rhinite, d'une rhinite allergique et d'une rhinosinusite chronique, éventuellement en combinaison avec *Polyposis nasi,* l'agent thérapeutique en association contenant en outre du dexpanthénol et/ou en particulier l'agent thérapeutique en association se présentant sous forme d'une formulation liquide, en particulier d'une formulation en aérosol, de préférence sous forme d'un spray nasal.

12. Agent thérapeutique en association pour l'utilisation selon l'une des revendications 1 à 8 pour prévenir ou réduire les pertes d'efficacité par des effets d'accoutumance et/ou d'habituation, en particulier avec congestion nasale permanente et/ou avec diminution de l'écoulement, l'agent thérapeutique en association contenant au moins un stéroïde, de préférence un corticostéroïde pouvant être administré par voie topique, de préférence pouvant être administré par voie nasale, en particulier un glucocorticostéroïde, et/ou l'agent thérapeutique en association se présentant sous forme d'une formulation liquide, en particulier d'une formulation en aérosol, de préférence sous forme d'un spray nasal.

13. Dispositif d'administration pour administration intranasale, en particulier sous forme d'un récipient comportant un dispositif compte-gouttes ou aérosol, contenant un agent thérapeutique en association selon l'une des revendications précédentes.

14. Dispositif pour administration selon la revendication 13,
le dispositif pour administration comportant un dispositif aérosol, pour expulser d'une manière uniforme la composition en une quantité par bouffée comprise dans la plage de 20 µl à 300 µl, en particulier dans la plage de 30 µl à 200 µl, de préférence dans la plage de 40 µl à 100 µl, ou
le dispositif pour administration comprenant un dispositif goutte-à-goutte pour l'expulsion uniforme de la composition en une quantité par goutte comprise dans la plage de 10 µl à 200 µl, en particulier dans la plage de 20 µl à 150 µl, de préférence dans la plage de 15 µl à 100 µl.
